# EUROPEAN PATENT APPLICATION

(11) **EP 4 471 126 A1**
(43) Date of publication of application: **04.12.2024**
(21) Application number: 23749723.5
(22) Date of filing: 30.01.2023
(51) Int. Cl.: C12N 5/077, C12N 5/071

(54) **LUNG MESENCHYMAL CELLS AND METHOD FOR PRODUCING LUNG MESENCHYMAL CELLS**

(30) Priority: 01.02.2022 JP 2022014212
(71) Applicant: Kyoto University, Kyoto-shi, Kyoto 606-8501 (JP)
(72) Inventor: GOTOH, Shimpei, Kyoto-shi, Kyoto 606-8501 (JP); TAMAI, Koji, Kyoto-shi, Kyoto 606-8501 (JP)
(74) Representative: J A Kemp LLP
(86) International application number: PCT/JP2023/002943
(87) International publication number: WO 2023/149407

(57) **Abstract**

The present disclosure provides a method for producing lung mesenchymal cells that can be used as feeder cells in induction of alveolar epithelial cells. A method for producing lung mesenchymal cells of the present disclosure includes culturing mesodermal cells in a presence of a mesenchymal cell-inducing factor, KGF, and FGF 10 so as to induce differentiation of the mesodermal cells into lung mesenchymal cells.

## Description

### TECHNICAL FIELD

The present invention relates to a method for producing lung mesenchymal cells and lung mesenchymal cells.

### BACKGROUND ART

When producing alveolar organoids using pluripotent stem cells, the alveolar organoids can be produced by co-culturing lung progenitor cells derived from the pluripotent stem cells and human fetal lung fibroblasts (HFLFs) (Non Patent Literatures 1 and 2). However, while HFLFs are allogeneic cells, they are not autologous cells. Thus, when the obtained alveolar organoids are transplanted, there is a problem of graft rejection. Also, HFLFs are difficult to obtain and there is an ethical problem because HFLFs are fetal-derived cells.

### Citation List

### Non Patent Literature

Non Patent Literature 1: Gotoh, Shimpei et al. "Generation of alveolar epithelial spheroids via isolated progenitor cells from human pluripotent stem cells." Stem cell reports vol. 3,3 (2014): 394-403. doi:10.1016/j.stemcr.2014.07.005
Non Patent Literature 2: Yamamoto, Yuki et al. "Long-term expansion of alveolar stem cells derived from human iPS cells in organoids." Nature methods vol. 14,11 (2017): 1097-1106. doi: 10.103 8/nmeth.4448

### SUMMARY OF INVENTION

### Technical Problem

In order to solve these problems, the inventors have developed a method for producing alveolar organoids without using HFLFs. However, in the method for producing alveolar organoids, there was a problem that type II alveolar epithelial cells were mainly induced and type I alveolar epithelial cells were difficult to be induced. This suggested that HFLFs were important as feeder cells in producing the alveolar organoids. Therefore, a method for inducing mesenchymal cells that function as feeder cells as well as HFLFs is needed.

With the foregoing in mind, an object of the present disclosure is to provide a method for producing lung mesenchymal cells that can be used as feeder cells in induction of alveolar epithelial cells.

### Solution to Problem

In order to achieve the above object, the present disclosure provides a method for producing lung mesenchymal cells, including: culturing mesodermal cells in a presence of a mesenchymal cell-inducing factor, KGF, and FGF10 so as to induce differentiation of the mesodermal cells into lung mesenchymal cells.

The presnet disclosure also provides a cell population including mesenchymal cells (hereinafter, also referred to as the "cell population"), including: lung mesenchymal cells that express at least one factor selected from the group consisting of RSPO2 and RSPO3.

The present disclosure also provides a cell population including mesenchymal cells, the cell population including: lung mesenchymal cells that express at least one transcription factor selected from the group consisting of Forkhead box protein F1 (FOXF1), Transcription factor 21 (TCF21), T-Box Transcription Factor 4 (TBX4), and Odd-Skipped Related Transcription Factor (OSR1).

The present disclosure also provides a method for producing lung epithelial cells and/or airway epithelial cells (hereinafter, also referred to as the "production method"), including: culturing lung progenitor cells in a presence of lung mesenchymal cells so as to induce differentiation of the lung progenitor cells into alveolar epithelial cells and/or airway epithelial cells, wherein the lung mesenchymal cells are the lung mesenchymal cells obtained by the method for producing lung mesenchymal cells according to the present disclosure or the cell population according to the present disclosure.

The present disclosure also provides a pharmaceutical composition including: the lung mesenchymal cells obtained by the method for producing lung mesenchymal cells according to the present disclosure or the cell population according to the present disclosure.

### Advantageous Effects of Invention

According to the present invention, it is possible to provide a method for producing lung mesenchymal cells that can be used as feeder cells in induction of alveolar epithelial cells.

### BRIEF DESCRIPTION OF DRAWINGS

[FIG. 1] FIG. 1 shows schematic diagrams showing the outline of the method for inducing lung mesenchymal cells and lung progenitor cells and a method forming alveolar organoids in Example 1.
[FIG. 2] FIG. 2 shows photographs of phase difference images showing differentiation states of cells after culturing and a stained image with oil red O in Example 1.
[FIG. 3] FIG. 3 shows graphs showing the flow cytometry analysis in Example 1.
[FIG. 4] FIG. 4 shows graphs showing the gene expressions of cells in each culture stage in Example 1.
[FIG. 5] FIG. 5 shows photographs showing fluorescence images of cells on day 7 of culture in Example 1.
[FIG. 6] FIG. 6 shows graphs showing the results of examination of alveolar organoids in Example 1.
[FIG. 7] FIG. 7 shows photographs and graphs showing the expressions of various cell markers in alveolar organoids in Example 1.
[FIG. 8] FIG. 8 is a schematic diagram showing an outline of an assay system in Example 2.
[FIG. 9] FIG. 9 shows photographs and graphs relating to the expressions of iMES markers in Example 2.
[FIG. 10] FIG. 10 shows graphs and photographs relating to the organoid formation ability in Example 2.
[FIG. 11] FIG. 11shows diagrams showing the analysis results of RNA-Seq in Example 2.
[FIG. 12] FIG. 12 is a view showing the relative expression levels of Wnt ligands in Example 2.
[FIG. 13] FIG. 13 shows a diagram showing the culture method and graphs and photographs showing the analysis results of SFTPC-GFP positive cells in Example 2.
[FIG. 14] FIG. 14 shows graphs showing the results of cluster analysis of mesenchymal cells in Example 2.
[FIG. 15] FIG. 15 shows a diagram, graphs, and photographs showing the passage results of type II alveolar epithelial cells in Example 3.
[FIG. 16] FIG. 16 shows graphs showing the cluster analysis results of the scRNA-seq analysis in Example 4.
[FIG. 17] FIG. 17 shows graphs showing the results showing the ligand-receptor interaction in Example 4.
[FIG. 18] FIG. 18 shows graphs showing the results of SFTPC-GFP positive cells/EPCAM positive cells in Example 5.

### DESCRIPTION OF EMBODIM ENTS

### <Definition>

As used herein, the term "marker" refers to a nucleic acid, a gene, a polypeptide, or a protein that is expressed to a different extent in a subject cell. When the marker is a positive marker, the different extent refers to increased expression as compared to that in undifferentiated cells. When the marker is a negative marker, the different extent refers to reduced expression as compared to that in undifferentiated cells.

As used herein, the term "positive (+)", "positive", or "express" means that a cell expresses a detectable marker. The term "positive (+)" typically means that the signal detected by an analytical method, such as flow cytometry of detecting by using an antigen-antibody reaction, is higher than that in the case of a negative control reaction using a negative control cell that does not express the antigen or an antibody that does not react with the antigen. The term "do not express" means that, when the expression level of the marker gene in the reference sample is compared with the expression level of the marker gene in the subject sample by RT-PCR or the like, an increased expression level of the marker gene is observed in the subject sample. The expression level is an expression level corrected by an internal standard gene (for example, a β-actin gene). When the subject sample is a cell or a cell population derived from pluripotent cells, induced pluripotent stem cells (iPS cells) can be used as the reference sample.

As used herein, the term "negative (-)", "negative", or "do not express" means that a cell does not express a detectable marker. The term "negative (-)" typically means that the signal detected by an analytical method, such as flow cytometry of detecting by using an antigen-antibody reaction, is equivalent to or lower than that in the case of a negative control reaction using a negative control cell that does not express the antigen or an antibody that does not react with the antigen. The term "express" means that, when the expression level of the marker gene in the reference sample is compared with the expression level of the marker gene in the subject sample by RT-PCR or the like, a reduced expression level of the marker gene is observed in the subject sample. The expression level is an expression level corrected by an internal standard gene (for example, a β-actin gene). When the subject sample is a cell or a cell population derived from pluripotent cells, induced pluripotent stem cells (iPS cells) can be used as the reference sample.

As used herein, the "pluripotent cells" refer to cells that are capable of being differentiated into ectodermal cells, mesodermal cells, and endodermal cells. If the pluripotent cells are capable of self-renewal, the pluripotent cells can also be referred to as pluripotent stem cells.

As used herein, the "mesodermal cells" refer to cells that are destined to be capable of being differentiated into connective tissues, such as bone, cartilage, blood vessels, and lymphatic vessels; and muscle tissues, and are cells that express mesodermal cell markers, such as Neural cell adhesion molecule (NCAM), Platelet Derived Growth Factor Receptor α (PDGFRα), Kinase Insert Domain Receptor (KDR), ISL1, NKX2-5 and/or OSR1, preferably cells that express NCAM, PDGFRα and/or KDR, and more preferably cells that express NCAM and/or PDGFRα.

As used herein, the "definitive endoderm (DE) cells" refer to, if there is an embryologically appropriate stimulus, cells that are destined to be capable of being differentiated into the thymus; digestive organs, such as the stomach, intestines, liver; respiratory organs, such as the trachea, bronchi, and lungs; and urinary organs, such as the bladder and urethra, and are cells that express SRY (sex determining region Y)-box 17 (SOX17) and Forkhead box protein A2 (FOXA2).

As used herein, the "anterior foregut endoderm (AFE) cells" (also referred to as "anterior foregut cells") refer to, if there is an embryologically appropriate stimulus, cells that are destined to be capable of being differentiated into the thymus; and respiratory organs, such as the trachea, bronchi, and lungs, and are cells that express SOX2, SOX17, and FOXA2.

As used herein, the "ventral anterior foregut endoderm (VAFE) cells" (also referred to as "ventral anterior foregut cells") refer to, if there is an embryologically appropriate stimulus, cells that are destined to be capable of being differentiated into the thyroid and lung, and are cells that express NKX2.1, GATA-binding factor 6 (GATA6), and Homeodomain-only protein (HOPX).

As used herein, the "mesenchymal cells" are cells derived from mesodermal cells, refer to, if there is an embryologically appropriate stimulus, cells that are destined to be capable of being differentiated into connective tissues, such as bone, cartilage, blood vessels, and lymphatic vessels, are cells that express mesenchymal cell markers, such as Vimentin (VIM), Thy-1 Cell Surface Antigen, CD90 (THY1), PDGFRα, Collagen Type I Alpha 1 Chain (COL1A1), NCAM, and/or KDR, and are preferably cells that expresses VIM, THY1, and/or COL1A1.

As used herein, the "lung mesenchymal cells" are cells derived from mesodermal cells, refer to, if there is an embryologically appropriate stimulus, cells that are destined to be capable of being differentiated into connective tissue of the lung, and are cells that express Forkhead box protein F1 (FOXF1), Transcription factor 21 (TCF21) and/or T-Box Transcription Factor 4 (TBX4) in addition to the mesenchymal cell markers. When the lung mesenchymal cells express fibroblast markers (e.g., Neural cell adhesion molecule (NCAM), Adipose differentiation-related protein (ADRP), and/or Collagen, type I, alpha 1 (COL1A1), and Actin alpha 2 (ACTA2)), the lung mesenchymal cells can also be referred to as lung fibroblasts.

As used herein, the "lung progenitor cells" refer to cells that are destined to be capable of being differentiated into alveolar epithelial cells and/or airway epithelial cells, if there is an embryologically appropriate stimulus. The lung progenitor cells are cells expressing carboxypeptidase M (CPM), NK2 homeobox 1 (NKX2.1 or NKX2-1), SRY-box 9 (SRY (sex determining region Y)-box 9, SOX9), SRY-box 2 (SRY (sex determining region Y)-box 2, SOX2), and/or forkhead box protein 2A (FOXA2). The lung progenitor cells are preferably CPM and/or NKX2.1 positive cells.

As used herein, the "alveolar epithelial cells" refer to epithelial cells present in the alveolus of the lung. Examples of the alveolar epithelial cells include type I alveolar epithelial cells and type II alveolar epithelial progenitor cells.

As used herein, the "type I alveolar epithelial cells" refer to histologically squamous epithelial cells, and are cells expressing Podoplanin (PDPN), Advanced Glycosylation End-Product Specific Receptor (AGER), Caveolin 1 (CAV1), HOP Homeobox (HOPX), and/or Aquaporin 5 (AQP5).

As used herein, the "type II alveolar epithelial cells" refer to epithelial cells that produce pulmonary surfactant proteins such as Surfactant protein C (SFTPC), Surfactant protein B (SFTPB), and the like, and are cells expressing Surfactant protein C (SFTPC), Surfactant protein B (SFTPB), ATP-binding cassette sub-family A member 3 (ABCA3), Lysosome-associated membrane glycoprotein 3 (DCLAMP), and/or Sodium-dependent phosphate transport protein 2B (SLC34A2).

As used herein, the "cell population" refers to a collection of cells including a desired cell and being formed of one or more cells. In the cell population, the proportion of the desired cells within all of the cells (also referred to as "purity") can be quantified, for example, as the proportion of cells that express one or more markers that are to be expressed by the desired cells. The purity is, for example, the proportion in living cells. The purity can be measured by, for example, flow cytometry, immunohistochemistry, in situ hybridization, RT-PCR, single-cell analysis, or the like. The purity of the desired cell in the cell population is, for example, 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more.

As used herein, the term "isolated" means identified and separated, or a state of being identified and separated, and/or identified and harvested from a component in a natural state, or a state of being harvested from a component in a natural state. The "isolation" can be carried out, for example, by obtaining with at least one purification.

As used herein, the term "enrichment" means increasing the content ratio of the subject cells or a state in which the content ratio of the subject cells is increased compared to that of before the treatment. The enrichment can also be referred to as condensation. The enrichment does not include, for example, a culture.

As used herein, "proteins" or "polypeptides" refer to polymers composed of unmodified amino acids (natural amino acids), modified amino acids, and/or artificial amino acids.

As used herein, "nucleic acid molecules" or "nucleic acids" refer to polymers of deoxyribonucleotides (DNA), ribonucleotides (RNA), and/or modified nucleotides. The nucleic acid molecule may be a single-stranded nucleic acid molecule or a double-stranded nucleic acid molecule.

As used herein, the term "subject" refers to animals or cells, tissues, or organs derived from animals, and is used in a sense that includes, in particular, humans. The animal means human and non-human animals. Examples of the non-human animal include mammals such as mice, rats, rabbits, dogs, cats, cows, horses, pigs, monkeys, dolphins, and sea lions.

As used herein, the term "treatment" refers to therapeutic treatment and/or prophylactic treatment. Also, the term "treatment" means the treatment, cure, prevention, suppression, amelioration, or improvement of a disease, condition, or disorder; or stopping, suppression, reduction, or delay of the progression of a disease, condition, or disorder. As used herein, the term "prevention" refers to a decrease in the possibility of developing a disease or condition, or a delay in the offset of a disease or condition. The term "treatment" may be, for example, treatment of a patient who develops a target disease, or treatment of a model animal with a target disease.

The sequence information of the proteins or nucleic acids (e.g., DNA or RNA) encoding the proteins described herein are available from Protein Data Bank, UniPort, Genbank, or the like.

The present disclosure is described below in more detail with reference to illustrative examples. The present disclosure, however, is not limited by the following description. In addition, the descriptions in the present disclosure can be referred to each other unless otherwise specified. In the present specification, the expression "** to **" is used to include numerical values or physical values before and after "to". Also, in the present specification, the expression "A and/or B" includes "A only", "B only", and "both A and B".

### <Method for Producing Lung Mesenchymal Cells>

In one aspect, the present disclosure provides a method for producing lung mesenchymal cells or a method for producing lung mesenchymal cells that can be used to form alveolar organoids. The method for producing lung mesenchymal cells of the present disclosure includes culturing mesodermal cells in the presence of a mesenchymal cell-inducing factor and KGF and/or FGF10 to induce differentiation into lung mesenchymal cells. According to the method for producing lung mesenchymal cells of the present disclosure, it is possible to provide lung mesenchymal cells that can be used to form alveolar organoids. In addition, when the alveolar organoids are formed using the lung mesenchymal cells obtained by the method for producing lung mesenchymal cells of the present disclosure, the alveolar organoids include, for example, type I alveolar epithelial cells, type II alveolar epithelial cells, and airway epithelial cells such as airway ciliated epithelial cells. Therefore, according to the method for producing lung mesenchymal cells of the present disclosure, for example, lung mesenchymal cells as feeder cells capable of being replaced with HFLFs can be provided.

In the method for producing lung mesenchymal cells of the present disclosure, the mesodermal cells used for inducing the lung mesenchymal cells can be derived from, for example, pluripotent cells. Thus, the method for producing lung mesenchymal cells of the present disclosure may induce, prior to inducing the lung mesenchymal cells from the mesodermal cells, differentiating the mesodermal cells from the pluripotent cells. In this case, the method for producing lung mesenchymal cells of the present disclosure includes, for example, culturing pluripotent cells in the presence of a mesoderm-inducing factor to induce differentiation into the mesodermal cells (first induction).

In the first induction, for example, the pluripotent cells are cultured in a medium containing the mesoderm-inducing factor to be differentiated into cells that express the mesodermal cell markers, i.e., mesodermal cells. That is, the culture is carried out in a state where the pluripotent cells are in contact with the mesoderm-inducing factor, thereby differentiating into mesodermal cells. Regarding the inducing of the mesodermal cells from the pluripotent cells, for example, References 1 to 4 below can be referenced. Specifically, in the first induction, for example, the mesodermal cells can be induced from the pluripotent cells by culturing using a GSK3β inhibitor, activin A, and/or BMP4 as the mesoderm-inducing factor. One type or two or more types, for example, of the mesoderm-inducing factors may be used. When one type of the mesoderm-inducing factor is used, the mesoderm-inducing factor is preferably the GSK3β inhibitor. When two or more types of the mesoderm-inducing factors are used in combination, the mesoderm-inducing factor may be, for example, a combination of the GSK3β inhibitor and activin A and/or BMP4; a combination of the GSK3β inhibitor and activin A and BMP4; and the like. When the mesoderm-inducing factor is a peptide or protein, the mesoderm-inducing factor is, for example, a peptide or protein derived from an animal species that is different from or the same as an animal species from which the pluripotent cell is derived. In the first induction, Lefty may be used instead of activin A. In the first induction, BMP2, BMP6, and/or BMP7 may be used instead of BMP4.
Reference 1: Han, L., Chaturvedi et al. "Single cell transcriptomics identifies a signaling network coordinating endoderm and mesoderm diversification during foregut organogenesis" Nat Commun, 2020, 11, 4158, which is herein incorporated by reference.
Reference 2: Kishimoto, K. et al, "Bidirectional Wnt signaling between endoderm and mesoderm confers tracheal identity in mouse and human cells" Nat Commun, 2020, 11, 4159, which is herein incorporated by reference.
Reference 3: Loh, K.M. et al., "Mapping the Pairwise Choices Leading from Pluripotency to Human Bone, Heart, and Other Mesoderm Cell Types" Cell, 2016, 166, 451-467, which is herein incorporated by reference.
Reference 4: Xi, H. et al. "In Vivo Human Somitogenesis Guides Somite Development from hPSCs" Cell Rep, 2017, 18, 1573-1585, which is herein incorporated by reference.

Examples of the pluripotent cells include totipotent stem cells such as induced pluripotent stem cells (iPS cells) and embryonic stem cells (ES cells); and pluripotent stem cells, such as tissue stem cells or somatic stem cells such as hematopoietic stem cells, neural stem cells, and mesenchymal stem cells.

As ES cells, for example, human embryonic stem cell lines such as H1, H7, and H9 (available from WiCell Research Institute) can be used. The ES cells may be prepared, for example, by culturing a cell mass isolated from an animal blastocyst. In particular, regarding the method for inducing ES cells, Reference 5 below can be referenced.
Reference 5: Thomson JA et al., "Embryonic stem cell lines derived from human blastocysts.", Science, 1998, vol. 282, pages 1145-1147, which is herein incorporated by reference.

As the iPS cells, 201B7 (available from RIKEN BRC), 604A1 (available from iPS Laboratory, Kyoto University), and the like can be used. The iPS cells can be prepared, for example, by introducing reprogramming factors into the subject cells. Examples of the reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1, and specific examples thereof include a combination of Oct3/4, Sox2, Klf4, L-Myc, and Lin28.

The GSK3β inhibitor may be a substance that inhibits the kinase activity of the GSK3β protein (e.g., the ability to phosphorylate β-catenin), and specific examples thereof include indirubin derivatives such as BIO (GSK-3 β inhibitor IX: 6-bromodirubin 3'-oxime); maleimide derivatives such as SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione); phenyl α bromomethyl ketone compounds such as SK-3 β inhibitor VII (4-dibromoacetophenone); L803-mts (GSK-3β peptide inhibitor); cell membrane-permeable phosphorylated peptides such as Myr-N-GKEAPPAPPQSpP-NH2; and CHIR99021 (6-[2-[4-(2,4-dichlorophenyl)-5-(4-methyl-1H-imidazol-2-yl)pyrimidin-2-ylamino]ethylamino]pyridine-3-carbonitrile); and GSK3β protein expression inhibiting nucleic acid molecules (siRNA, shRNA, antisense, and the like). The GSK3β inhibitor is preferably CHIR99021 because it is highly selective for GSK3β. The GSK3β inhibitor is commercially available, for example, from Calbiochem and Biomol.

Activin A is a protein (SEQ ID NO: 49) encoded by the polynucleotide registered in NCBI under the accession NO: NM _002192.
Activin A (SEQ ID NO: 49)

MPLLWLRGFLLASCWIIVRSSPTPGSEGHSAAPDCPSCALAALPKDVPNSQPEMVEAVKK
HILNMLHLKKRPDVTQPVPKAALLNAIRKLHVGKVGENGYVEIEDDIGRRAEMNELME
QTSEIITFAESGTARKTLHFEISKEGSDLSVVERAEVWLFLKVPKANRTRTKVTIRLFQQQ
KHPQGSLDTGEEAEEVGLKGERSELLLSEKVVDARKSTWHVFPVSSSIQRLLDQGKSSL
DVRIACEQCQESGASLVLLGKKKKKEEEGEGKKKGGGEGGAGADEEKEQSHRPFLMLQ
ARQSEDHPHRRRRRGLECDGKVNICCKKQFFVSFKDIGWNDWIIAPSGYHANYCEGECP
SHIAGTSGSSLSFHSTVINHYRMRGHSPFANLKSCCVPTKLRPMSMLYYDDGQNIIKKDI
QNMIVEECGCS

A functional equivalent of activin A may be used as activin A. The functional equivalent is a substance capable of activating a SMAD2/3 signal through an activin receptor (ACVR1/2) as with activin A. Examples of the functional equivalent of activin A include Nodal and Lefty.

BMP4 is a protein encoded by the polynucleotide registered in NCBI under the accession NO: NM_001202, NM_001347914, NM_001347916, NM_130850, or NM_130851. As an example, BMP4 may be, for example, a protein consisting of an amino acid sequence represented by SEQ ID NO: 50 below.
BMP4 (SEQ ID NO: 50)

A functional equivalent of BMP4 may be used as BMP4. The functional equivalent is a substance capable of activating a SMAD1/5/8 signal through a BMP receptor (BMPR1/2) as with BMP4. Examples of the functional equivalent of BMP4 include BMP2, BMP6, and BMP7.

The concentration of the mesoderm-inducing factor in the first induction is not particularly limited, and may be an effective concentration at which each factor exhibits an inducing activity to mesodermal cells. Specifically, when CHIR99021, which is a GSK3β inhibitor, is used as the mesoderm-inducing factor, the concentration of CHIR99021 in the medium is, for example, 0.1 to 20 µmol/L. When activin A is used as the mesoderm-inducing factor, the concentration of activin A in the medium is, for example, 1 to 100 ng/mL. When BMP4 is used as the mesoderm-inducing factor, the concentration of BMP4 in the medium is 1 to 100 ng/mL.

The medium may be prepared using a medium used to culture animal cells as a basal medium. Examples of the basal medium include IMDM, Medium 199, Eagle's Minimum Essential Medium (EMEM), αMEM, Dulbecco's modified Eagle's Medium (DMEM), Ham's F12 medium, RPMI1640 medium, Fischer's medium, Neurobasal Medium (Thermo Fisher Scientific Inc.), stem cell medium (e.g., mTeSR-1 (STEMCELL Technologies), TeSR-E8 (STEMCELL Technologies), CDM-PVA, StemPRO hESC SFM (Life Technologies), E8 (Life Technologies)), and mixed media thereof. The medium may be a medium supplemented with serum or without serum. The medium may include, for example, serum substitutes such as albumin, transferrin, Knockout Serum Replacement (KSR) (serum substitute for ES cell culture), N2 supplement (Invitrogen), B27 supplement (Invitrogen), fatty acids, insulin, collagen precursors, trace elements, 2-mercaptoethanol, 3'-thiolglycerol, and the like. In addition, the medium may contain additives such as lipids, amino acids, L-glutamines, and Glutamax (Invitrogen), non-essential amino acids, vitamins, growth factors, low molecular weight compounds, antibiotics, antioxidants, pyruvate, buffers, mineral salts, and the like. When the growth culture is carried out using said structure, the medium is preferably a medium for stem cell culture supplemented with glutamic acid and an antibiotic.

The culture period of the first induction may be a period during which the mesodermal cells can be differentiated, and is, for example, 1 to 7 days, 1 to 5 days, or 2 to 4 days.

As the culture conditions of the first induction, for example, normal conditions of cell culture can be adopted. As a specific example, the culture temperature is, for example, 25 to 40°C, 30 to 40°C, or about 37°C. The concentration of carbon dioxide in the culture is 1 to 10%, 3 to 7%, or about 5%. The culture is carried out, for example, in a wet environment.

In the first induction, the differentiation of the mesodermal cells can be detected, for example, by the expressions of the mesodermal cell markers and/or the loss of the expressions of the pluripotent cell markers.

Examples of the mesodermal cell marker include NCAM, PDGFRα, KDR, ISL1, NKX2-5, and/or OSR1. The mesodermal cell marker is preferably NCAM, PDGFRα, and/or KDR, and more preferably NCAM and/or PDGFRα, or NCAM and PDGFRα.

Examples of the pluripotent stem cell marker include ABCG2, Cripto, FOXD3, Connexin43, Connexin45, Oct4, Sox2, Nanog, hTERT, UTF1, ZFP42, SSEA-3, SSEA-4, TRA-1-60, and TRA-1-81.

After the first induction, the content ratio (lower limit) of the mesodermal cells within all of the cells (cell population) after the induction is, for example, 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more based on the number of cells. The content ratio (upper limit) is, for example, 100% or less, 99% or less, 98% or less, 97% or less, 96% or less, 95% or less, 94% or less, 93% or less, 92% or less, 91% or less, 90% or less, 85% or less, 80% or less, 75% or less, 70% or less, 65% or less, 60% or less, 55% or less, or 50% or less based on the number of cells. The numerical range of the content ratio may be, for example, any combination of the lower limit and the upper limit. As a specific example, in the first induction, when the culture is carried out for 3 days, the content ratio is, for example, 30 to 60%. The content ratio is lowered, for example, by shortening the number of culture days in the first induction. On the other hand, the content ratio is increased, for example, by increasing the number of culture days in the first induction.

Next, in the method for producing lung mesenchymal cells of the present disclosure, the mesodermal cells are cultured in the presence of a mesenchymal cell-inducing factor and KGF and FGF10 to induce differentiation into lung mesenchymal cells (second induction).

In the second induction, for example, the mesodermal cells are cultured in a medium containing a mesenchymal cell-inducing factor to be differentiated into cells that express the mesenchymal cell markers, i.e., mesenchymal cells such as lung mesenchymal cells. In the second induction, for example, the mesenchymal cells are differentiated, for example, by culturing the mesodermal cells in a state of being in contact with the mesenchymal cell-inducing factor. Specifically, in the second induction, for example, the lung mesenchymal cells can be induced from the mesodermal cells by culturing the mesodermal cells using activin A, FGF2, BMP4, retinoic acid (RA), PDGFbb (platelet-derived growth factor bb), a Wnt inducing agent, and/or a GSK3β inhibitor and KGF(FGF7) and/or FGF10 as the mesenchymal cell-inducing factor. In the second induction, in the method for inducing mesenchymal cells, the lung mesenchymal cells can be induced by allowing KGF and FGF10 to coexist. Regarding the method for inducing mesenchymal cells, for example, reference can be made to References 6 and 7 below. One type or two or more types, for example, of the mesenchymal cell-inducing factors may be used. When one type of the mesenchymal cell-inducing factor is used, the mesenchymal cell-inducing factor is preferably BMP4 or FGF2. When two or more types of the mesenchymal cell-inducing factors are used in combination, the mesenchymal cell-inducing factors may be, for example, a combination of activin A, FGF2, and BMP4; a combination of retinoic acid, BMP4, and a Wnt inhibitor and/or a GSK3β inhibitor (Reference 6); a combination of FGF2 and PDGFbb (Reference 7); and the like. When the mesenchymal cell-inducing factor is a peptide or protein, the mesoderm-inducing factor is, for example, a peptide or protein derived from an animal species that is different from or the same as an animal spices from which the mesodermal cell is derived. KGF and FGF10 are, for example, peptides or proteins derived from an animal species that is different from or the same as an animal spices from which the mesodermal cell is derived. In the second induction, FGF1 may be used instead of FGF2. In the second induction, FGF3 and/or FGF22 may be used instead of KGF and FGF10 (References 8 and 9). Regarding the Wnt inhibitor and the GSK3β inhibitor, reference can be made to the examples below. Reference 6: Han, Lu et al. "Single cell transcriptomics identifies a signaling network coordinating endoderm and mesoderm diversification during foregut organogenesis." Nature communications vol. 11,1 4158. 27 Aug. 2020, doi:10.1038/s41467-020-17968-x, which is herein incorporated by reference.
Reference 7: Takebe, Takanori et al. "Massive and Reproducible Production of Liver Buds Entirely from Human Pluripotent Stem Cells." Cell reports vol. 21,10 (2017): 2661-2670. doi:10.1016/j.celrep.2017.11.005, which is herein incorporated by reference.
Reference 8: Morichika Konishi, et al., "Regulation of Biological Function by Extracellular Secretory Factor FGF21", Internet.
<https://seikagaku.jbsoc.or.jp/10.14952/SEIKAGAKU.2016.880086/index.html>, which is herein incorporated by reference.
Reference 9: Hui, Qi et al. "FGF Family: From Drug Development to Clinical Application." International journal of molecular sciences vol. 19,7 1875. 26 Jun. 2018, doi: 10.3390/ijms19071875, which is herein incorporated by reference.

FGF2 is a protein (SEQ ID NO: 51) encoded by the polynucleotide registered in NCBI under the accession NO: NM_002006. FGF2 may be in an activated form upon cleavage by a protease.
FGF2 (SEQ ID NO: 51)

MVGVGGGDVEDVTPRPGGCQISGRGARGCNGIPGAAAWEAALPRRRPRRHPSVNPRSR

AAGSPRTRGRRTEERPSGSRLGDRGRGRALPGGRLGGRGRGRAPERVGGRGRGRGTAA
PRAAPAARGSRPGPAGTMAAGSITTLPALPEDGGSGAFPPGHFKDPKRLYCKNGGFFLRI
HPDGRVDGVREKSDPHIKLQLQAEERGVVSIKGVCANRYLAMKEDGRLLASKCVTDEC
FFFERLESNNYNTYRSRKYTSWYVALKRTGQYKLGSKTGPGQKAILFLPMSAKS

A functional equivalent of FGF2 may be used as FGF2. The functional equivalent is a substance capable of activating Ras-Raf through a FGF receptor (FGFR1 or FGFR4) as with FGF2. Examples of the functional equivalent of FGF2 include FGF1 and the like belonging to FGF1 subfamily as with FGF2.

KGF is a protein encoded by the polynucleotide registered in NCBI under accession NO: NM_002009. KGF may be in an activated form upon cleavage by a protease.

A functional equivalent of KGF may be used as KGF (FGF7). The functional equivalent is a substance capable of activating Ras-Raf through a FGF receptor (FGFR2b, FGFR1b, or the like) as with KGF. Examples of the functional equivalent of KGF include FGF3, FGF10, FGF22, and the like belonging to FGF7 subfamily as with KGF. KGF may be in an activated form upon cleavage by a protease.

FGF10 is a protein encoded by the polynucleotide registered in NCBI under accession NO: NM_004465. FGF10 may be in an activated form upon cleavage by a protease.

A functional equivalent of FGF10 may be used as FGF10. The functional equivalent is a substance capable of activating Ras-Raf through a FGF receptor (FGFR2b, FGFR1b) as with FGF10. Examples of the functional equivalent of FGF10 include KGF, FGF3, FGF22, and the like belonging to FGF7 subfamily as with FGF10. FGF10 may be in an activated form upon cleavage by a protease.

The concentration of the mesenchymal cell-inducing factor in the second induction is not particularly limited, and may be an effective concentration at which each factor exhibits an inducing activity to mesenchymal cells. Specifically, when activin A is used as a mesenchymal cell-inducing factor, the concentration of activin A in the medium is, for example, 0.01 to 1000 ng/mL, 0.1 to 100 ng/mL, or 0.2 to 10 ng/mL. When FGF2 is used as a mesenchymal cell-inducing factor, the concentration of FGF2 in the medium is, for example, 0.1 to 1000 ng/mL, 1 to 100 ng/mL, or 2 to 50 ng/mL. When BMP4 is used as a mesenchymal cell-inducing factor, the concentration of BMP4 in the medium is, for example, 0.1 to 1000 ng/mL, 1 to 100 ng/mL, or 2 to 50 ng/mL.

The concentration of KGF and FGF10 in the second induction is not particularly limited, and may be an effective concentration at which each factor exhibits an inducing activity to lung mesenchymal cells. As a specific example, the concentration of KGF in the medium is, for example, 0.1 to 1000 ng/mL, 1 to 100 ng/mL, or 2 to 50 ng/mL. The concentration of FGF10 in the medium is, for example, 0.1 to 1000 ng/mL, 1 to 100 ng/mL, or 2 to 50 ng/mL.

Regarding the medium, reference can be made to the description as to the medium in the first induction. The medium used in the second induction may be the same as or different from the medium used in the first induction.

The culture period of the second induction may be a period during which the lung mesenchymal cells can be differentiated, and is, for example, 1 to 9 days, 3 to 7 days, or 4 to 6 days.

Regarding the culture conditions of the second induction, reference can be made to the description as to the culture conditions of the first induction. The culture conditions of the second induction may be the same as or different from those of the first induction.

In the second induction, the differentiation of the lung mesenchymal cells can be detected, for example, by the expressions of the lung mesenchymal cell markers and/or the loss of the expressions of the mesodermal cell markers.

Examples of the mesenchymal cell marker include PDGFRα, KDR, ISL1, NKX2-5, VIM, COL1A1, FOXF1, and/or TCF21, and FOXF1 and TCF21 are preferable.

The mesodermal cell marker may be, for example, T-box transcription factor T (TBXT).

After the second induction, the content ratio (lower limit) of the lung mesenchymal cells in the whole cells (cell population) after the inducing is, for example, 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more based on the number of cells. The content ratio (upper limit) is, for example, 100% or less, 99% or less, 98% or less, 97% or less, 96% or less, 95% or less, 94% or less, 93% or less, 92% or less, 91% or less, 90% or less, 85% or less, 80% or less, 75% or less, 70% or less, 65% or less, 60% or less, 55% or less, or 50% or less based on the number of cells. The numerical range of the content ratio may be, for example, any combination of the lower limit and the upper limit. In the second cultivation, when the culture is carried out for 3 days, the content ratio is, for example, 20 to 40%. In the second cultivation, when the culture is carried out for 7 days, the content ratio is, for example, 60 to 90%. The content ratio is lowered, for example, by shortening the number of culture days in the second induction. On the other hand, the content ratio is increased, for example, by increasing the number of culture days in the first induction.

The method for producing lung mesenchymal cells of the present disclosure may enrich the lung mesenchymal cells after the second induction. Thus, the method for producing lung mesenchymal cells of the present disclosure can improve the efficiency of inducing type II alveolar epithelial cells when alveolar organoids are formed using, for example, a cell population including the obtained lung mesenchymal cells and the lung progenitor cells to be described below. In this case, the method for producing lung mesenchymal cells of the present disclosure includes enriching lung mesenchymal cells from a cell population induced from the mesodermal cells after the second induction (enrichment).

The enrichment of the lung mesenchymal cells can be carried out, for example, by using, as an indicator, a marker (positive marker) that is expressed in the lung mesenchymal cells but not expressed in other cells or expressed in low in other cells in the cell population, or a marker (negative marker) that is not expressed in the lung mesenchymal cells but expressed in other cells or expressed in high in other cells in the cell population. Preferably, the positive marker and the negative marker are expressed on the cell surface. Examples of the positive marker include PDGFRα and KDR, VIM, THY1, and NCAM. Examples of the negative marker include EpCAM and E-Cadherin. The enrichment may be carried out using a combination of two or more markers. The enrichment is carried out, for example, on the lung mesenchymal cells using the negative marker in order to suppress the generation of signal transduction through the marker.

The enrichment can be carried out, for example, by using an antibody against the positive marker and/or an antibody against the negative marker after harvesting the cell population after the second induction, using an automated magnetic cell separator (e.g., autoMACS), a magnetic cell separator (e.g., MACS), a closed magnetic cell separator (e.g., Prodigy), or a cell sorter (e.g., FACS).

After the enrichment, the content ratio (lower limit) of the positive marker-positive lung mesenchymal cells in the cell population after the enrichment is, for example, 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more based on the number of cells. The content ratio (upper limit) of the positive marker-positive lung mesenchymal cells is, for example, 100% or less, 99% or less, 98% or less, 97% or less, 96% or less, 95% or less, 94% or less, 93% or less, 92% or less, 91% or less, 90% or less, 85% or less, 80% or less, 75% or less, 70% or less, 65% or less, 60% or less, 55% or less, or 50% or less based on the number of cells. The numerical range of the content ratio of the positive marker-positive lung mesenchymal cells may be, for example, any combination of the lower limit and the upper limit. Further, after the enrichment, the content ratio (lower limit) of the negative marker-negative lung mesenchymal cells in the cell population after the enrichment is, for example, 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more based on the number of cells. The content ratio (upper limit) of the negative marker-negative lung mesenchymal cells is, for example, 100% or less, 99% or less, 98% or less, 97% or less, 96% or less, 95% or less, 94% or less, 93% or less, 92% or less, 91% or less, 90% or less, 85% or less, 80% or less, 75% or less, 70% or less, 65% or less, 60% or less, 55% or less, or 50% or less based on the number of cells. The numerical range of the content ratio of the negative marker-negative lung mesenchymal cells may be, for example, any combination of the lower limit and the upper limit.

As a specifice example, when the marker used for the enrichment is EpCAM, the content ratio (lower limit) of the EpCAM negative lung mesenchymal cells in the cell population after the enrichment is, for example, 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more based on the number of cells. The content ratio (upper limit) of the EpCAM negative lung mesenchymal cells is, for example, 100% or less, 99% or less, 98% or less, 97% or less, 96% or less, 95% or less, 94% or less, 93% or less, 92% or less, 91% or less, or 90% or less based on the number of cells. The numerical range of the content ratio of the EpCAM negative lung mesenchymal cells may be, for example, any combination of the lower limit and the upper limit.

The EpCAM negative lung mesenchymal cells may be, for example, PDGFRα, KDR, VIM, and/or THY1 positive. The content ratio (lower limit) of the PDGFRα positive and KDR positive lung mesenchymal cells in the EpCAM negative lung mesenchymal cells is, for example, 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99 % or more based on the number of cells. The content ratio (upper limit) of the PDGFRα positive and KDR positive lung mesenchymal cells is, for example, 100% or less, 99% or less, 98% or less, 97% or less, 96% or less, 95% or less, 94% or less, 93% or less, 92% or less, 91% or less, or 90% or less based on the number of cells. The numerical range of the content ratio of the PDGFRα positive and KDR positive lung mesenchymal cells may be, for example, any combination of the lower limit and the upper limit. The content ratio (lower limit) of the VIM positive and THY1 positive lung mesenchymal cells in the EpCAM negative lung mesenchymal cells is, for example, 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more based on the number of cells. The content ratio (upper limit) of the VIM positive and THY1 positive lung mesenchymal cells is, for example, 100% or less, 99% or less, 98% or less, 97% or less, 96% or less, 95% or less, 94% or less, 93% or less, 92% or less, 91% or less, or 90% or less based on the number of cells. The numerical range of the content ratio of the VIM positive and THY1 positive lung mesenchymal cells may be, for example, any combination of the lower limit and the upper limit.

The lung mesenchymal cells obtained by the method for producing lung mesenchymal cells of the present disclosure may be, for example, identified by expressions of various nucleic acids and proteins.

The lung mesenchymal cell expresses, for example, R-Spondin 2 (RSPO2) and/or R-Spondin 3 (RSPO3). Also, the lung mesenchymal cell, for example, does not express WNT2.The lung mesenchymal cell expresses, for example, RSPO2 and/or RSPO3 and does not express WNT2. Since the lung mesenchymal cell expresses RSPO2 and/or RSPO3, for example, type II alveolar epithelial cells can be induced when alveolar organoids are formed using a cell population including the obtained lung mesenchymal cells and the lung progenitor cells to be described below.

The content ratio (lower limit) of RSPO2 positive lung mesenchymal cells in the EpCAM negative lung mesenchymal cells is, for example, 5% or more, 10% or more, 15% or more, 20% or more, 25% or more, or 30% or more based on the number of cells. The content ratio (upper limit) of RSPO2 positive lung mesenchymal cells in the EpCAM negative lung mesenchymal cells is, for example, 50% or less, 45% or less, 40% or less, 35% or less, 30% or less, or 25% or less based on the number of cells. The numerical range of the content ratio of the RSPO2 positive lung mesenchymal cells may be, for example, any combination of the lower limit and the upper limit. As a specific example, in the second cultivation, when the culture is carried out for 7 days, the content ratio is, for example, 20 to 40%.

In the EpCAM negative lung mesenchymal cells, RSPO2 is expressed in particular in, for example, STC1 (Stanniocalcin-1) positive lung mesenchymal cells. Thus, RSPO2 positive cells can be enriched, for example, by using STC1. The content ratio (lower limit) of RSPO2 positive lung mesenchymal cells in the EpCAM negative STC1 positive lung mesenchymal cells is, for example, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more based on the number of cells. The content ratio (upper limit) of RSPO2 positive lung mesenchymal cells is, for example, 100% or less, 99% or less, 98% or less, 97% or less, 96% or less, 95% or less, 94% or less, 93% or less, 92% or less, 91% or less, or 90% or less based on the number of cells. The numerical range of the content ratio of the RSPO2 positive lung mesenchymal cells may be, for example, any combination of the lower limit and the upper limit. As a specific example, in the second cultivation, when the culture is carried out for 7 days, the content ratio is, for example, 80% or more.

The content ratio (lower limit) of RSPO3 positive lung mesenchymal cells in the EpCAM negative lung mesenchymal cells is, for example, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more based on the number of cells. The content ratio (upper limit) of RSPO3 positive lung mesenchymal cells in the EpCAM negative lung mesenchymal cells is, for example, 100% or less, 99% or less, 98% or less, 97% or less, 96% or less, 95% or less, 94% or less, 93% or less, 92% or less, 91% or less, or 90% or less based on the number of cells. The numerical range of the content ratio of the RSPO3 positive lung mesenchymal cells may be, for example, any combination of the lower limit and the upper limit. As a specific example, in the second cultivation, when the culture is carried out for 7 days, the content ratio is, for example, 70 to 90%.

The content ratio (lower limit) of Wnt2 negative lung mesenchymal cells in the EpCAM negative lung mesenchymal cells is, for example, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more based on the number of cells. The content ratio (upper limit) of Wnt2 negative lung mesenchymal cells in the EpCAM negative lung mesenchymal cells is, for example, 100% or less, 99% or less, 98% or less, 97% or less, 96% or less, 95% or less, 94% or less, 93% or less, 92% or less, 91% or less, or 90% or less based on the number of cells. The numerical range of the content ratio of the Wnt2 negative lung mesenchymal cells may be, for example, any combination of the lower limit and the upper limit. As a specific example, in the second cultivation, when the culture is carried out for 7 days, the content ratio is, for example, 95% or more.

The lung mesenchymal cell, for example, expresses a transcription factor selected from the group consisting of Forkhead box protein F1 (FOXF1), Transcription factor 21 (TCF21), T-Box Transcription Factor 4 (TBX4), and Odd-Skipped Related Transcription Factor (OSR1). The lung mesenchymal cell, for example, may express one type or two or more types of the transcription factors, or may express all types of the transcription factors. The lung mesenchymal cell does not express TBXT, for example, as a transcription factor. The lung mesenchymal cell expresses, for example, a transcription factor selected from the group consisting of FOXF1, TCF21, TBX4, and OSR1 and does not express TBXT. The lung mesenchymal cell may, for example, further express the fibroblast marker to be described below and/or be mesenchymal cell marker positive.

The lung mesenchymal cell, for example, expresses a fibroblast marker selected from the group consisting of NCAM, ADRP, COL1A1, and ACTA2. The lung mesenchymal cell is preferably a cell that expresses NCAM, ADRP, and/or COL1A1; NCAM, ADRP, and COL1A1. The lung mesenchymal cell, for example, may express one type or two or more types of the fibroblast markers or may express all types of the fibroblast markers

The lung mesenchymal cell, for example, is positive for a mesenchymal cell marker selected from the group consisting of Vimentin (VIM), Thy-1 Cell Surface Antigen, CD90 (THY1), Platelet Derived Growth Factor Receptor α (PDGFRα), and Kinase Insert Domain Receptor (KDR). The lung mesenchymal cell is preferably a cell that expresses VIM, THY1, and/or COL1A1; or VIM, THY1, and COL1A1. The lung mesenchymal cell, for example, may be positive for one type or two or more types of the mesenchymal cell markers or all types of the mesenchymal cell markers.

The lung mesenchymal cells can induce, for example, epithelial cells constituting alveoli from the lung progenitor cells to be described below. Thus, the lung mesenchymal cells can induce, for example, in an alveolar organoid formation assay by co-culturing with the progenitor cells, type I alveolar epithelial cells and/or type II alveolar epithelial cells from the lung progenitor cells. The alveolar organoid formation assay can be carried out in the same manner as that in Example 1(3) to be described below. Examples of the alveolar epithelial cells include type I alveolar epithelial cells and type II alveolar epithelial cells. The lung mesenchymal cells may, for example, be capable of inducing the type I alveolar epithelial cells or the type II alveolar epithelial cells from the lung progenitor cells, or may be capable of inducing the type I alveolar epithelial cells and the type II alveolar epithelial cells from the lung progenitor cells.

For example, in the alveolar organoid formation assay, an EpCAM positive cell population having a proportion (lower limit) of SFTPC positive cells of 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more can be induced from the lung progenitor cells. The upper limit of the proportion may be, for example, 100% or less, 99% or less, 98% or less, 97% or less, 96% or less, 95% or less, 94% or less, 93% or less, 92% or less, 91% or less, 90% or less, 85% or less, 80% or less, 75% or less, 70% or less, 65% or less, 60% or less, or 55% or less. The numerical range of the proportion may be, for example, any combination of the lower limit and the upper limit. As a specific example, in the second cultivation, when the lung mesenchymal cells and the lung progenitor cells are cultured for 14 days, the proportion of SFTPC positive cells is 20 to 70%.

According to the method for producing lung mesenchymal cells of the present disclosure, for example, alveolar epithelial cells can be induced when co-cultured with the lung progenitor cells. It is expected that the lung mesenchymal cells can be suitably used, for example, as cells used for tissue regeneration of the lung.

### <Cell Population>

In another aspect, the present disclosure provides a cell population including lung mesenchymal cells that can also be used to form alveolar organoids. The cell population including the mesenchymal cells of the present disclosure includes lung mesenchymal cells that express RSPO2 and/or RSPO3.

The lung mesenchymal cells of the present disclosure may be identified, for example, by the expressions of various nucleic acids and proteins described in the description as to the lung mesenchymal cells obtained by the method for producing lung mesenchymal cells of the present disclosure, i.e., the lung mesenchymal cells after the second induction or after the enrichment.

### <Method for Producing Alveolar Epithelial Cells>

In another aspect, the present disclosure provides a method for producing alveolar epithelial cells using the lung mesenchymal cells. The method for producing alveolar epithelial cells of the present disclosure includes culturing lung progenitor cells in a presence of lung mesenchymal cells to induce differentiation into alveolar epithelial cells, wherein the lung mesenchymal cells are the lung mesenchymal cells obtained by the method for producing lung mesenchymal cells according to the present disclosure and/or the cell population including mesenchymal cells according to present disclosure.

In the method for producing alveolar epithelial cells of the present disclosure, the lung progenitor cells used to induce the alveolar epithelial cells can be induced from progenitor cells of lung progenitor cells, such as pluripotent cells. Therefore, the method for producing alveolar epithelial cells of the present disclosure may induce the lung progenitor cells from the progenitor cells of the lung progenitor cells prior to the induction of the alveolar epithelial cells. In this case, the method for producing alveolar epithelial cells of the present disclosure includes, for example, culturing in the presence of a lung progenitor cell-inducing factor to induce differentiation into the lung progenitor cells (third induction).

In the third induction, for example, the progenitor cells of the lung progenitor cells are cultured in a medium containing a lung progenitor cell-inducing factor to be differentiated into lung progenitor cells that express the lung progenitor cell markers, that is, the progenitor cells of the lung progenitor cells are cultured in a state of being in contact with the lung progenitor cell-inducing factor to be differentiated into the lung progenitor cells. Regarding the inducing the lung progenitor cells from the progenitor cells of the lung progenitor cells, reference can be made to, for example, a method for inducing alveolar epithelial progenitor cells described in WO 2014/168264, a method for inducing lung airway progenitor cells described in WO 2019/217429, a method for isolating lung progenitor cells described in U.S. Pat. No. 10,386,368, or a method for inducing NKX2-1 lung progenitor cells described in Reference 10.
Reference 10: Hawkins et al., J Clin Invest. 2017 Jun 1;127(6):2277-2294. doi: 10.1172/JCI89950, which is herein incorporated by reference.

Examples of the progenitor cells of the lung progenitor cells include ventral anterior foregut endoderm cells, anterior foregut endoderm cells, and/or definitive endoderm cells. The lung progenitor cells and the progenitor cells of the lung progenitor cells can be induced from the pluripotent cells or the pluripotent stem cells. Therefore, the lung progenitor cells and the progenitor cells of the lung progenitor cells are preferably progenitor cells induced from the pluripotent cells or the pluripotent stem cells. Examples of the pluripotent stem cells include totipotent stem cells such as induced pluripotent stem cells (iPS cells) and embryonic stem cells (ES cells); tissue stem cells, such as hematopoietic stem cells, neural stem cells, and mesenchymal stem cells; and pluripotent stem cells, such as somatic stem cells.

The cell population obtained in the third induction is a cell population including the lung progenitor cells and/or the progenitor cells of the lung progenitor cells. In the method for producing alveolar epithelial cells of the present disclosure, the obtained cell population may be used as it is, or the lung progenitor cells and/or the progenitor cells of the lung progenitor cells may be isolated from the obtained cell population to use. When isolating the lung progenitor cells, the lung progenitor cells can be isolated, for example, based on the expressions of CPM, NKX2.1, SOX9, SOX2, and/or FOXA2. Preferably, the lung progenitor cells are isolated as CPM positive cells using CPM, which is a cell surface-marker.

Next, in the method for producing alveolar epithelial cells of the present disclosure, the lung progenitor cells are cultured in the presence of the lung mesenchymal cells to induce differentiation into alveolar epithelial cells (fourth induction).

In the fourth induction, for example, the lung progenitor cells are cultured in a medium containing a lung mesenchymal cell-inducing factor to be differentiated into alveolar epithelial cells that express the alveolar epithelial cell marker, that is, the lung progenitor cells are brought into contact with the lung progenitor cell-inducing factor to differentiate the lung progenitor cells. In the fourth induction, the lung progenitor cells may be cultured in the presence of the lung mesenchymal cells and an alveolar epithelial cell-inducing factor to induce differentiation into alveolar epithelial cells.

The alveolar epithelial cell-inducing factor may be determined according to the type of the alveolar epithelial cells to be induced. When the alveolar epithelial cells are type I alveolar epithelial cells, the alveolar epithelial cell-inducing factors are type I alveolar epithelial cell-inducing factors, and specifically, for example, the Wnt inducing agent. One type or two or more types of the inducing factors may be used. Preferably two or more types of the inducing factors are used and more preferably all of the inducing factors are used.

The Wnt inducing agent is a substance that induces a Wnt signal. Examples of the Wnt inducing agent include IWP2 (N-(6-methyl-2-benzothiazolyl)-2-(3,4,6,7-tetrahydro-4-oxo-3-phenylthieno3,2-dpyrimidin-2-yl)thio), Dickkopf-related protein 1 (DKK1), XAV939 (3,5,7,8-tetrahydro-2-[4-(trifluoromethyl)phenyl]-4H-thiopyrano[4,3-d]pyrimidin-4-one); and Wnt protein expression inducing nucleic acid molecules (siRNA, shRNA, antisense, and the like), and XAV939 is preferable.

The concentration of the Wnt inducing agent in the medium is, for example, 1 nmol/L to 50 µmol/L, 10 nmol/L to 40 µmol/L, 50 nmol/L to 30 µmol/L, 100 nmol/L to 25 µmol/L, and 500 nmol/L to 20 µmol/L.

Regarding the medium, reference can be made to the description as to the medium in the first induction.

The number of culture days in the inducing type I alveolar epithelial cells can be determined according to a period during which the type I alveolar epithelial cells are induced. The lower limit of the number of culture days may be, for example, 4 or more days, 5 or more days, 6 or more days, 7 or more days, 8 or more days, 9 or more days, 10 or more days, 11 or more days, 12 or more days, or more. The upper limit of the number of culture days is, for example, 35 or less days, 30 or less days, 28 or less days, or 21 or less days.

Regarding the culture conditions of the inducing type I alveolar epithelial cells, reference can be made to the description as to the culture conditions of the first induction.

When the alveolar cells are type II alveolar epithelial cells, the alveolar epithelial cell-inducing factors are type II alveolar epithelial cell-inducing factors, and specific examples thereof include a steroid agent, a cAMP derivative, a phosphodiesterase inhibitor, KGF, a GSK3β inhibitor, a TGFβ inhibitor, a ROCK inhibitor and/or FGF10. One type or two or more types of the inducing factors may be used. Preferably two or more types of the inducing factors are used and more preferably, a combination of the steroidal agent, the cAMP derivative, the phosphodiesterase inhibitor, and KGF is used. Regarding the KGF, the GSK3β inhibitor, and the FGF10, reference can be made to the above description.

The steroid agent is a steroid anti-inflammatory drug. Examples of the steroid agent include a glucocorticoid or a synthetic derivative thereof, and specific examples thereof include hydrocortisone, hydrocortisone succinate, prednisolone, methylprednisolone, methylprednisolone succinate, triamcinolone, triamcinolone acetonide, dexamethasone, and betamethasone, and dexamethasone or hydrocortisone is preferable.

The concentration of the steroid agent in the medium is, for example, 1 nmol/L to 100 µmol/L, 1 nmol/L to 50 µmol/L, 10 nmol/L to 40 µmol/L, 10 nmol/L to 30 µmol/L, 10 nmol/L to 25 µmol/L, and 10 nmol/L to 20 µmol/L.

The cAMP derivative is a compound that is cyclic AMP modified (added) with a substituent. Examples of the cAMP derivative include cyclic adenosine monophosphate (cAMP), 8-bromo cyclic adenosine monophosphate (8-Br-cAMP), 8-chloro cyclic adenosine monophosphate (8-Cl-cAMP), 8-(4-chlorophenylthio)cyclic adenosine monophosphate (8-CPT-cAMP), and dibutyryl cyclic adenosine monophosphate (DB-cAMP), and 8-Br-cAMP is preferable.

The concentration of the cAMP derivative in the medium is, for example, 1 nmol/L to 100 µmol/L, 1 nmol/L to 50 µmol/L, 10 nmol/L to 40 µmol/L, 50 nmol/L to 30 µmol/L, 100 nmol/L to 25 µmol/L, 500 nmol/L to 20 µmol/L.

The phosphodiesterase inhibitor is a compound that increases intracellular concentration of cAMP or cGMP by inhibiting phosphodiesterase (PDE). Examples of the phosphodiesterase inhibitor include 1,3-dimethylxanthine, 6,7-dimethoxy-1-(3,4-dimethoxybenzyl)isoquinoline, 4-f [3',4'-(methylenedioxy)benzyl]amino}-6-methoxyquinazoline, 8-methoxymethyl-3-isobutyl-1-methylxanthine, and 3-isobutyl-1-methylxanthine(IBMX), and 1,3-dimethylxanthine is preferable.

The concentration of the phosphodiesterase inhibitor in the medium is, for example, 1 nmol/L to 100 µmol/L, 1 nmol/L to 50 µmol/L, 10 nmol/L to 40 µmol/L, 50 nmol/L to 30 µmol/L, 50 nmol/L to 25 µmol/L, and 50 nmol/L to 20 µmol/L.

The TGFβ inhibitor is a substance that inhibits signal transduction through SMAD caused by binding of TGFβ to a receptor. Examples of the TGFβ inhibitor include a substance that inhibits binding to ALK family of TGFβ receptors, and a substance that inhibits phosphorylation of SMAD by the ALK family. Specific examples of the TGFβ inhibitor include Lefty-1 (NCBI accession NO: NM_010094 (mouse), NM_020997 (human), SB431542 (4-(4-(benzo[d][1,3]dioxol-5-yl)-5-(pyridine-2-yl)-1H-imidazol-2-yl)benzamide), SB202190 (4-(4-fluorophenyl)-2-(4-hydroxyphenyl)-5-(4-pyridyl)-1H-imidazole), SB505124 (2-(5-benzo1,3dioxol-5-yl-2-tert-butyl-3H-imidazol-4-yl)-6-methylpyridine), NPC30345, SD093, SD908, SD208 (Scios), LY2109761, LY364947, LY580276 (Lilly Research Laboratories), and A-83-01 (WO2009/146408), and SB431542 is preferable.

The concentration of the TGFβ inhibitor in the medium is, for example, 1 nmol/L to 50 µmol/L, 10 nmol/L to 40 µmol/L, 50 nmol/L to 30 µmol/L, 100 nmol/L to 25 µmol/L, or 500 nmol/L to 20 µmol/L, and is preferabLy, 1 nmol/L to 40 µmol/L.

The ROCK inhibitor is a substance that can inhibit the function of Rho kinase (ROCK). Examples of the ROCK inhibitor include Y-27632 ((+)-(R)-trans-4-(1-aminoethyl)-N-(4-pyridyl)cyclohexanecarboxamide dihydrochloride), Fasudil/HA1077 (5-(1,4-diazepane-1-sulfonyl)isoquinoline), H-1152 ((S)-(+)-2-methyl-1-[(4-methyl-5-isoquinolinyl)sulfonyl]homopiperazine), Wf-536 ((+)-(R)-4-(1-aminoethyl)-N-(4-pyridyl) benzamide), and ROCK protein expression inhibiting nucleic acid molecules (siRNA, shRNA, antisense, and the like) and the like, and Y-27632 is preferable.

The concentration of the ROCK inhibitor in the medium is, for example, 1 nmol/L to 50 µmol/L, 10 nmol/L to 40 µmol/L, 50 nmol/L to 30 µmol/L, 100 nmol/L to 25 µmol/L, 500 nmol/L to 20 µmol/L, or 750 nmol/L to 15 µmol/L, and is preferabLy 1 nmol/L to 40 µmol/L.

Regarding the medium, reference can be made to the description as to the medium in the first induction.

The number of culture days in the inducing type II alveolar epithelial cells can be determined according to a period during which the type II alveolar epithelial cells are induced. The lower limit of the number of culture days may be, for example, 2 or more days, 4 or more days, 5 or more days, 6 or more days, 7 or more days, 8 or more days, 9 or more days, 10 or more days, 11 or more days, 12 or more days, 13 or more days, 14 or more days, 15 or more days, or more. The upper limit of the number of culture days is, for example, 35 or less days, 30 or less days, 28 or less days, or 21 or less days.

Regarding the culture conditions of the inducing type I alveolar epithelial cells, reference can be made to the description as to the culture conditions of the first induction.

In the fourth induction, the differentiation of the alveolar epithelial cells can be detected, for example, by the expressions of the alveolar epithelial cell markers and/or the loss of the expressions of the lung progenitor cell markers. The alveolar epithelial cells may be, for example, a cell population including the type I alveolar epithelial cells or the type II alveolar epithelial cells, or a cell population including the type I alveolar epithelial cells and the type II alveolar epithelial cells.

When the alveolar epithelial cells are type I alveolar epithelial cells, examples of the alveolar epithelial cell marker include PDPN, AGER, CAV1, HOPX, and AQP5. When the alveolar epithelial cells are type II alveolar epithelial cells, examples of the alveolar epithelial cell marker include SFTPC, SFTPB, ABCA3, DCLAMP, and SLC34A2.

According to the method for producing alveolar epithelial cells of the present disclosure, for example, the alveolar epithelial cells can be induced as organoids containing alveolar epithelial cells.

### <Method for Maintenance-Culturing and/or Expand-Culturing Alveolar Epithelial Cells>

In another aspect, the present disclosure provides a method capable of maintenance-culturing and/or expand-culturing type II alveolar epithelial cells. The method for maintenance-culturing and/or expand-culturing type II alveolar epithelial cells of the present disclosure includes culturing type II alveolar epithelial cells in the presence of lung mesenchymal cells to maintain or expand the cells (cultivation), wherein the lung mesenchymal cells are the lung mesenchymal cells obtained by the method for producing lung mesenchymal cells of the present disclosure and/or the cell population including mesenchymal cells of the present disclosure.

The type II alveolar epithelial cells are known to function as lung tissue stem cells. Thus, in the cultivation, the type II alveolar epithelial cells can be maintained and/or expanded by, for example, inducing self-renewal (growth) of the type II alveolar epithelial cells or self-renewal (growth) and differentiation of the type II alveolar epithelial cells.

In the cultivation, for example, the lung progenitor cells are cultured in a medium containing the lung mesenchymal cells to maintain or grow the lung progenitor cells that express the lung progenitor cell markers, or to maintain or grow the lung progenitor cells that express the lung progenitor cell markers as well as differentiate into alveolar epithelial cells that express the alveolar epithelial cell markers. In the cultivation, the lung progenitor cells may be cultured in the presence of the lung mesenchymal cells and an alveolar epithelial cell-inducing factor to maintain or expand the lung progenitor cells.

The alveolar epithelial cell-inducing factor can be determined according to the type of alveolar epithelial cells to be induced, and reference can be made to the description as to the method for producing alveolar epithelial cells of the present disclosure.

### <Pharmaceutical Composition>

In another aspect, the present disclosure provides a pharmaceutical composition including lung mesenchymal cells. The pharmaceutical composition of the present disclosure includes the lung mesenchymal cells of the present disclosure and a pharmaceutically acceptable carrier.

The method for administering a pharmaceutical composition of the present disclosure is, for example, intravenous administration. The dosage form of the pharmaceutical composition of the present disclosure is, for example, an injection. In the case of the injection, the number of lung mesenchymal cells contained in the injection is, for example, 1×10⁶ or more. The pharmaceutical composition may include a pharmaceutically acceptable carrier. Examples of the carrier include physiological saline, phosphate buffered saline (PBS), cell preservation solutions, cell culture solutions, hydrogels, extracellular matrices, and cryopreservation solutions.

The pharmaceutical composition of the present disclosure can be suitably used, for example, for the treatment of lung diseases.

### <Medium>

In another aspect, the present disclosure provides a medium for use in inducing lung mesenchymal cells from mesodermal cells. The medium for use in inducing lung mesenchymal cells from mesodermal cells of the present disclosure includes a medium, a mesenchymal cell-inducing factor, KGF, and FGF10.

Regarding the type, combination, and concentration of the mesenchymal cell-inducing factor, KGF, and FGF10 in the medium, for example, reference can be made to the description as to the method for producing lung mesenchymal cells of the present disclosure.

### <Kit>

In another aspect, the present disclosure provides a kit for use in inducing lung mesenchymal cells from mesodermal cells. The kit for use in inducing lung mesenchymal cells from mesodermal cells of the present disclosure includes a mesenchymal cell-inducing factor, KGF, and FGF10.

Regarding the type and combination of the mesenchymal cell-inducing factor, KGF, and FGF10 in the kit, for example, reference can be made to the description as to the method for producing lung mesenchymal cells of the present disclosure. The content of the mesenchymal cell-inducing factor, KGF, and FGF10 can be determined so as to be the concentration of each factor in the description as to the method for producing mesenchymal cells of the present disclosure, for example, when added to a predetermined amount of medium.

### Examples

Next, examples of the present disclosure will be described. The present disclosure, however, is not limited by the following examples. Commercially available reagents were used based on their protocols unless otherwise indicated.

### [Example 1]

It was examined whether lung mesenchymal cells can be induced by the production method of the present disclosure, and that alveolar organoids can be formed by co-culturing the lung mesenchymal cells and the lung progenitor cells.

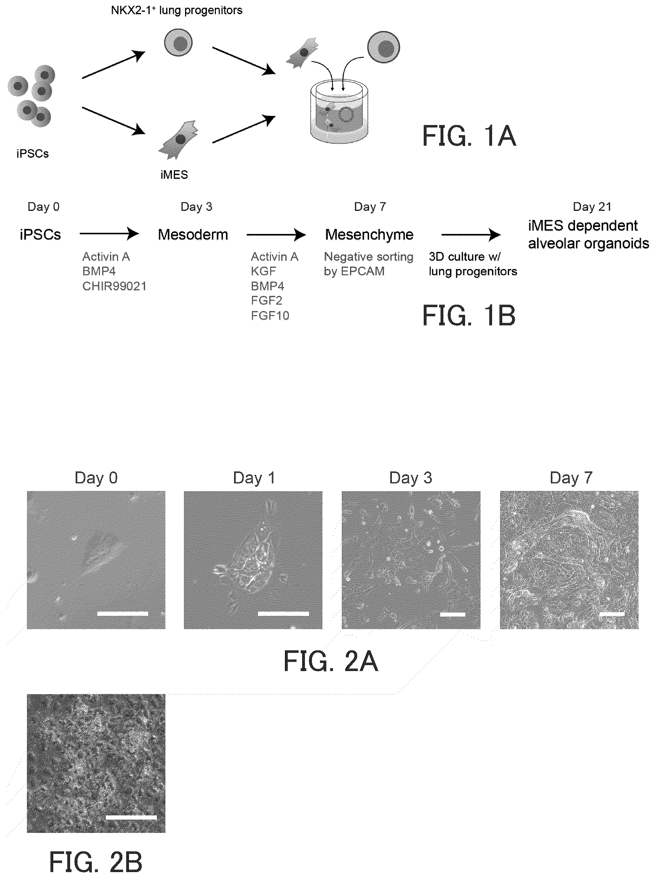

The outline of the method for inducing lung mesenchymal cells and lung progenitor cells and forming alveolar organoids is shown in FIG. 1.

### (1) Induction of Lung Mesenchymal Cells

As shown in (B) of FIG. 1, the lung mesenchymal cells were induced from human-derived iPS cells (iPSCs). Specifically, undifferentiated human iPSCs that had been subcultured were washed with D-PBS (Nacalai Tesque, Inc., Cat. No.: 14249-24), and then incubated at 37°C for 20 minutes in the presence of a protease (Accutase, Innovative Cell Technologies, Inc., Cat. No.: AT-104) to dissociate iPSCs into single cells. mTeSR Plus (STEMCELL Technologies, Cat. No.: ST-05825 or ST-100-0276) in equal amounts was added to neutralize the protease, and then the cell suspension containing iPSCs was centrifuged to remove the supernatant. The harvested cell suspension was then seeded in a 6-well plate at a cell density of 8 to 15×10⁴ using mTeSR Plus that contains iMatrix-511 (0. 25 µg/day) and 10 µmol/L Y-27632 (LC Laboratories (Cat. No.: Y-5301). Next, on day 0 of culture, the medium was replaced with STEMPRO^{®}-34 (Thermo Fisher Scientific Inc., Cat. No.: 10639011) supplemented with 150 ng/mL activin A (API Corporation, Cat. No.: GF-001), 50 ng/mL BMP4 (R&D Systems, Inc., Cat. No.: 314-BP), 1.5 µmol/L CHIR99021 (Axon Medchem, Cat. No.: Axon1386), GLUTAMAX^{®} (Thermo Fisher Scientific Inc., Cat. No.: 35050061), and 50 U/mL penicillin/streptomyci. Then, on day 2 of culture, the medium was replaced with the same medium, thereby inducing mesodermal cells from iPSCs. Hereinafter, the differentiation state of the cells was observed using a phase-contrast microscopy.

On day 3 of culture, the medium was replaced with STEMPRO^{®} -34 supplemented with 30 ng/mL activin A, 10 ng/mL KGF (ProSpec, Cat. No.: CYT-219), 25 ng/mL BMP4, 10 ng/mL bFGF (DS Pharma Biomedical Co., Ltd., Cat. No.: KHFGF001), 10 ng/mL FGF10, Glutamax, and 50 U/mL penicillin/streptomycin. On day 5 of culture, the medium was replaced with the same medium. Then, on day 7 of culture, cells were peeled off from the plate by treating with TrypLE Select Enzyme (Thermo Fisher Scientific Inc. (Cat. No.: 12563029)) at 37°C for 10 minutes. The resulting suspension of cells was washed with 2% FBS-containing DMEM and then suspended in 1% BSA-containing PBS. Then, 1 µL of the antibody solution was added to 1×10⁶ /100 µL of cells, and the cells were incubated in the obtained mixture at room temperature (about 25°C, the same applies hereinafter) for 20 minutes. As the antibody in the antibody solution, an anti EPCAM antibody (Santa Cruz Biotechnology, Inc., Cat. No.: sc-66020/EBA-1) was used. Next, anti-mouse IgG microbeads (Miltenyi Biotec, Cat. No.: 130-048-401) were used as a secondary antibody to react with the mixture after the incubation, and then LD columns (Miltenyi Biotec, Cat. No.: 130-042-901) were used to negatively sort EPCAM negative cells from the obtained reaction solution according to the attached protocol. The harvested mesenchymal cells were then used as lung mesenchymal cells (iMESs) hereinafter.

The cells on day 7 of culture were stained with oil red O for granules. In addition, cells on day 0, day 1, day 3, and day 7 of culture were stained with Anti-EPCAM-FITC antibody (Miltenyi Biotec, Cat. No.: 130-080-301), Anti-NCAM-Alexa Fluor 647 antibody (BioLegend, Inc., Cat. No.: 362513), Anti-T-Alexa Fluor 488 antibody (R&D Systems, Inc., Cat. No.: IC2085G), Anti-KDR-BV421 antibody (BioLegend, Inc., Cat. No.: 393009), Anti-THY1 (CD90)-BV421 antibody (BioLegend, Inc., Cat. No.:328121), Anti-Vimentin-Alexa Fluor 647 antibody (Novus Biologicals, Cat. No.: NBP1-97670AF647), and Anti-PDGFRA-Alexa Fluor 647 antibody (Novus Biologicals, Cat. No.:562798). Specifically, a single-cell suspension was washed with 1% BSA-containing PBS and then stained with a primary antibody at 4°C for 15 minutes. After washing with 1% BSA-containing PBS, the cell suspension was stained with a secondary antibody at 4°C for 15 minutes, as required. After washing cells twice with 1% BSA-containing PBS, the cell suspension was stained with propidium iodide (PI). When performing intracellular staining, the cell suspension was fixed for 20 minutes using BD Cytofix/Cytoperm (BD Biosciences, Cat. No.:51-2090KZ), and then permeabilized for 20 minutes using BD Perm/Wash (BD Biosciences, Cat. No.:51-2091KZ). After the permeabilization, the cell suspension was washed twice with BD Perm/Wash, and then stained with a primary antibody at 4°C for 15 minutes. Next, the cell suspension after the staining was washed twice with BD Perm/Wash, and then stained with a secondary antibody at 4°C for 15 minutes. After washing twice with 1% BSA-containing PBS, cells were prepared with 1% BSA/PBS that contains no PI. The obtained stained samples were subjected to flow cytometry analysis using Melody (BD Biosciences). The results thereof are shown in FIGs. 2 to 3.

FIG. 2 shows photographs of phase difference images showing the differentiation state of cells after culturing and a stained image with oil red O. In FIG. 2, (A) shows phase difference images, and (B) shows a stained image with oil red O. Each scale bar in FIG. 2 represents 100 µm. In (A) of FIG. 2, the photographs are of, from the left, on day 0, day 1, day 3, and day 7 of culture. As shown in (A) of FIG. 2, while the cells are in the form of PSCs on day 0 of culture, the border of the cell mass became unclear on day 1, suggesting that differentiation started. These are presumed to be cells expressing TBXT in the EpCAM positive cell population to be described below. On day 3 of culture, it was observed that the cells became squamous and were differentiated into mesodermal cells. Then, on day 7 of culture, the cells became cells with many granules in the cytoplasm, and as shown in (B) of FIG. 2, these granules were stained with Oil Red O.

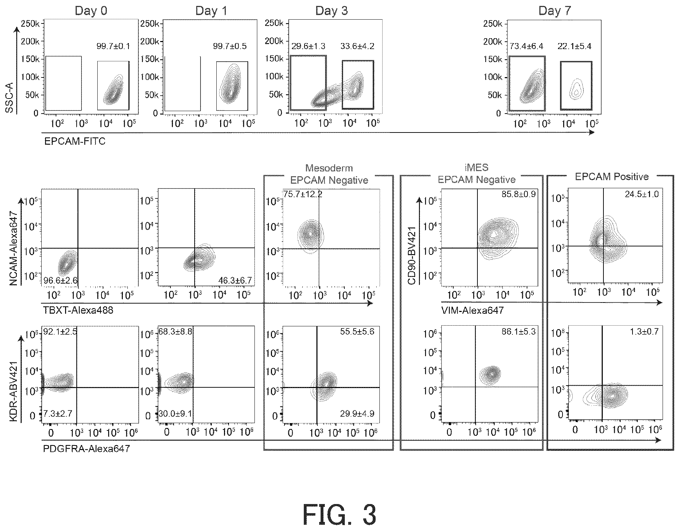

FIG. 3 shows graphs showing flow cytometry analysis. In FIG. 3, the graphs in the upper low show, from the left, the results on day 0, day 1, day 3, and day 7 of culture. Further, in FIG. 3, the graphs in the middle low and graphs in the lower low show, from the left, the results of the EpCAM negative cell populations on day 0, day 1, and day 3 of culture, and the EpCAM negative cell populations and the EpCAM positive cell populations on day 7 of culture. As shown in FIG. 3, cells positive for EpCAM and TBXT were observed on day 1 of culture, suggesting that the cells were differentiated into cells similar to the primitive streak developmentally. On day 3 of culture, the expressions of NCAM, PDGFRα, and KDR, which are mesodermal cell markers, were observed in the EpCAM negative cell population, showing that the cells were differentiated into mesodermal cells. On day 7 of culture, mesenchymal cells positive for VIM, THY1 (CD90), PDGFRα, and KDR, which are mesenchymal cell markers, were induced. These results showed that mesenchymal cells were induced even when KGF and FGF10 were added in the condition for inducing mesenchymal cells.

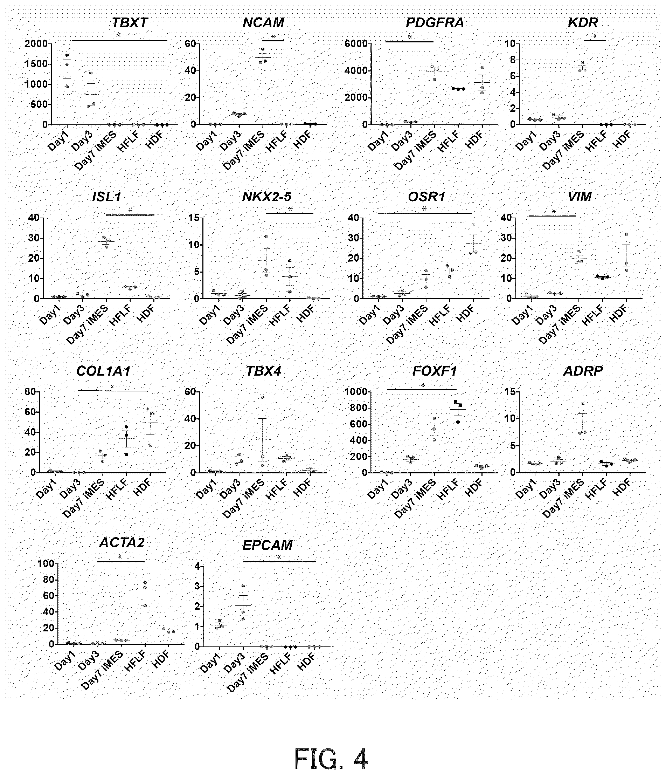

Next, the gene expression in each culture stage was examined using RT-qPCR. Specifically, the expressions of genes in cells on day 0 of culture and in cells on day 3 of culture, iMESs, HFLFs, and HDFs were examined. Extraction of total RNAs from the cells was performed using an RNA extraction kit (PureLink RNA mini kit, Thermo Fisher Scientific Inc., Cat. No.:12183020). Then, cDNA was prepared using reverse transcriptase (SUPERSCRIPT^{®} III reverse transiptase, Thermo Fisher Scientific Inc.) for a total RNA of 80 ng per sample. The obtained cDNA was amplified using an RT-PCR kit (Power SYBR Green PCR Master Mix, Applied Biosystems), and quantified using a QuantStudio 3 (Applied Biosystems). The expression level of each gene was normalized (standardized) using the β-actin gene as an internal standard gene. Furthermore, the expression level was quantified as the relative gene expression level to the gene expression level in the cells on day 0 of culture. The primer sets used for RT-qPCR are shown below in Table 1. The results thereof are shown in FIG. 4.

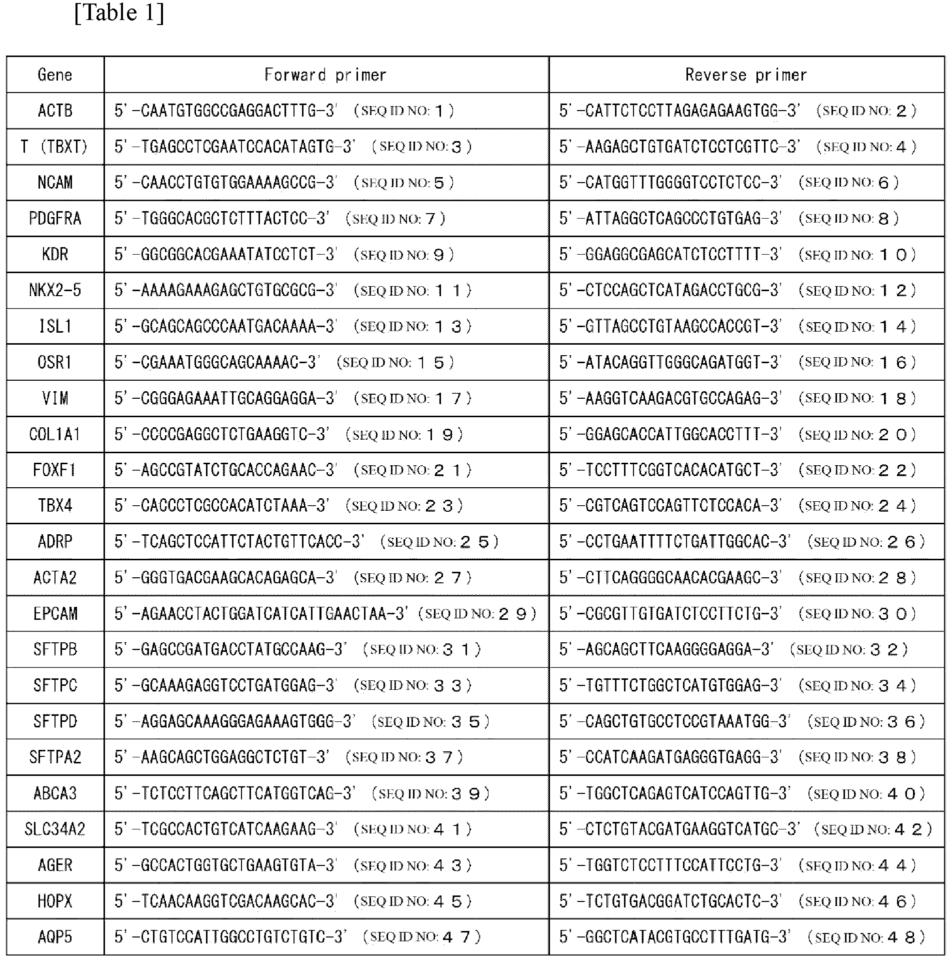

FIG. 4 shows graphs showing the gene expression of cells at each culture stage. In each graph in FIG. 4, the horizontal axis indicates the culture stage of cells, and the vertical axis indicates the relative expression level. As shown in FIG. 4, after the start of culture, TBXT and EPCAM were expressed in cells in the early stage of culture, but not in iMESs. In iMESs, the expressions of the fibroblast markers, VIM and COL1A1, were induced. In addition, the lung mesenchymal cell markers, FOXF1 and TBX4, were expressed in iMESs. In iMESs, increased expressions of NCAM, PDGFRα, KDR, ISL1, NKx2-5, OSR1, and ADRP were observed. These results showed that the iMESs induced by the method for producing lung mesenchymal cells of the present disclosure were lung mesenchymal cells. These results also showed that the iMESs could be distinguished from other cells using these markers.

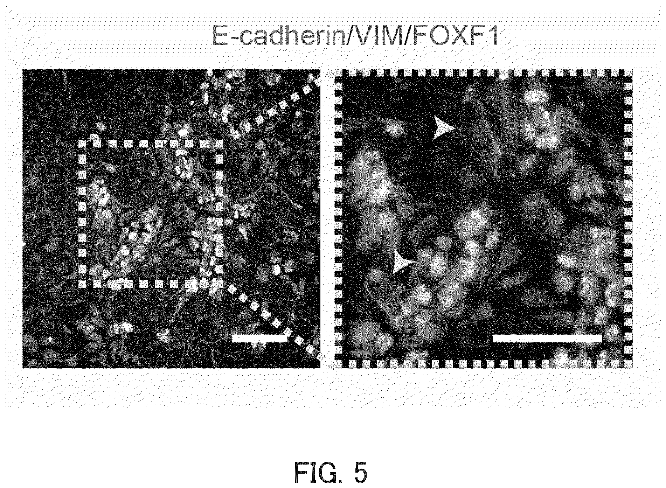

Next, the cells on day 7 of culture were fixed with 4% paraformaldehyde-containing PBS for 15 minutes, and then permeabilized with 0.2% TRITON^{®} X-100-containing PBS for 15 minutes. After the permeabilization, the primary antibody and the secondary antibody were used for staining as described above (Reference 11 below). As the primary antibody, Anti-E-Cadherin antibody (eBiosience, Cat No.: 14-3249), Anti-Vimentin antibody (Cell Signaling Technology, Inc., Cat No.: 49636), and Anti-FOXF1 antibody (R&D Systems, Inc., Cat No.: AF4798) were used. Further, as the secondary antibody, Anti-rat IgG Alexa Fluor 488 (Thermo Fisher Scientific Inc., Cat No.: A-21208), Anti-mouse IgG Alexa Fluor 546 (Thermo Fisher Scientific Inc., Cat No.: A-10036), and Anti-goat IgG Alexa Fluor 647 (Thermo Fisher Scientific Inc., Cat No.: A-21447) were used. The stained samples were observed using a fluorescent microscope (BZ-X710, Keyence). The results thereof are shown in FIG. 5.
Reference 11: Gotoh, S., Ito, I., Nagasaki, T., Yamamoto, Y, Konishi, S., Korogi, Y, Matsumoto, H., Muro, S., Hirai, T., Funato, M., et al. (2014). Generation of alveolar epithelial spheroids via isolated progenitor cells from human pluripotent stem cells. Stem Cell Reports 3, 394-403, which is herein incorporated by reference.

FIG. 5 shows photographs showing fluorescence images of cells on day 7 of culture. Each scale bar in FIG. 5 represents 100 µm. As shown in FIG. 5, E-cadherin positive cells were FOXF1 negative while E-cadherin negative cells were VIM and FOXF1 positive. These results showed that iMESs expressed VIM and FOXF1 on a protein-level basis.

### (2) Induction of Lung Progenitor Cells

Differentiation into lung progenitor cells derived from human iPSCs was carried out in the order according to Reference 11 and the following References 12-13. Specifically, undifferentiated human iPSCs were cultured on a Geltrex coated plate in the presence of a definitive endoderm induction medium for 6 days to be differentiated into definitive endoderm cells (Step1). For differentiation into definitive endoderm cells, a RPMI1640 medium (Nacalai Tesque, Inc., Cat. No.: 30264-56) that contains 100 ng/mL activin A, 1 µmol/L CHIR99021, 2% B27 supplement (Thermo Fisher Scientific Inc., Cat. No.: 17504-001), and 50 U/mL penicillin/streptomycin was used. Thereafter, each medium was changed every 2 days. As summarized in Table 2 below, in the differentiation into definitive endoderm cells, Y-27632 was added on day 0 of culture, and sodium butyrate (FUJIFILM Wako Pure Chemical Corporation, Cat. 193-015122) was added on day 1, day 2, and day 4 of culture.
Reference 12: Konishi, S., Gotoh, S., Tateishi, K., Yamamoto, Y, Korogi, Y, Nagasaki, T., Matsumoto, H., Muro, S., Hirai, T., Ito, I., et al. (2016). Directed Induction of Functional Multi-ciliated Cells in Proximal Airway Epithelial Spheroids from Human Pluripotent Stem Cells. Stem Cell Reports 6, 18-25, which is herein incorporated by reference.
Reference 13: Yamamoto, Y, Gotoh, S., Korogi, Y, Seki, M., Konishi, S., Ikeo, S., Sone, N., Nagasaki, T., Matsumoto, H., Muro, S., et al. (2017). Long-term expansion of alveolar stem cells derived from human iPS cells in organoids. Nat Methods 14, 1097-1106.

**[Table 2]**

| | Definitive endoderm induction medium (Step1) | Anteriorization medium (Step2) | Posteriorization medium (Step3) | CFKD preconditioning medium (Step4) |
|---|---|---|---|---|
| Basal medium | RPMI | DMEM / F12 | | |
| | B27 supplement (2%) | Glutamax | | |
| | Penicilin/ Streptomycin (50 U/ml) | B27 supplement (2%) | | |
| | | L-ascorbic acid (0.05 mg/ml) | | |
| | | Monothioglycerol (0.4 mmol/l) | | |
| | | Penicilin/Streptomycin (50 U/ml) | | |
| Compound/cytokine | Activin A (100 ng/ml) | Noggin (100 ng/ml) | ATRA (Varies depending on cell line) | CHIR99021 (3 µmol/l) |
| | CHIR99021 (1.0 µmol/l) | SB431542 (10 µmol/l) | B2-3: 0.05 µmol/l | FGF10 (10 ng/ml) |
| | Y-27632 (Day 0 of culture: 10 µmol/l) | | CHIR99021 (3 µM) | KGF (10 ng/ml) |
| | Sodium butyrate (NaB) (Day 1, day 2, and day 4 of culture) | | BMP4 (20 ng/ml) | DAPT (20 *µ*mol/l) |

The definitive endoderm cells were then cultured in an anteriorization medium on day 6 to day 10 of culture (Step2), followed by medium replacement with a posteriorization medium that contains BMP4 (20ng/mL) and ATRA (Sigma-Aldrich Co. LLC, Cat. No.: R2625) and CHIR99021 of given concentrations on day 10 of culture (Step3). When B2-3 line PSC was used, the optimal concentrations of ATRA and CHIR99021 were 0.05 to 0.5 µmol/L, respectively. On day 14 to day 21 of culture, cells after posteriorization were cultured in a CFKD preconditioning medium (Step4). Then, on day 21 of culture, NKX2-1 positive lung progenitor cells were isolated using mouse anti-human CPM (FUJIFILM Wako Pure Chemical Corporation, Cat. No.: 014-27501) and anti-mouse IgG-Alexa647 (Thermo Fisher Scientific Inc., Cat. No.: A-31571) for gating CPM positive cells in the same manner as that in Reference 13. Note that some lung progenitor cells are induced from PSCs (B2-3 line) expressing SFTPC-GFP, and when differentiated into alveolar epithelial cells, the expression of GFP is induced.

### (3) Alveolar Organoid Formation Assay

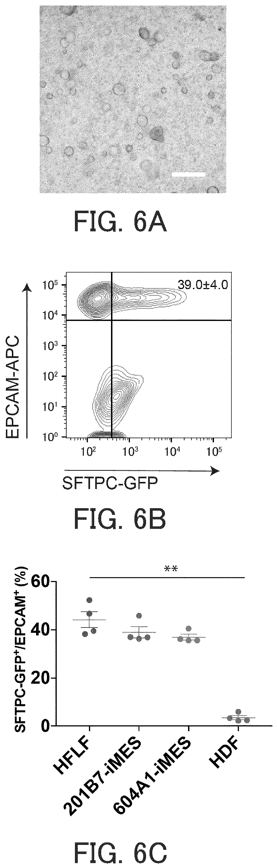

Alveolar organoids were prepared according to Reference 13 and the following Reference 14. 1.0×10⁴ CPM positive cells (derived from 201B7 line PSC), 5.0×10⁵ human fetal lung fibroblasts (HFLFs, DV Biologics LLC, Cat No.: PP002-F-1349), human pediatric dermal fibroblasts (HDFs, TIG120, available from National Institutes of Biomedical Innovation, Health and Nutrition), or iMESs were mixed in 100 µL of the alveolarization medium supplemented with Y-27632 (10 µmol/L) and 100 µL Matrigel (Corning Incorporated Incorporated, Cat. No.: 354230) summarized in Table 3 below. The resulting mixture was introduced onto a 12-well cell culture insert (Corning Incorporated, Cat. No.: 3513). Then, the cells were cultured for 14 days. The medium in the lower chamber was changed every 2 days. HFLFs were cultured in 10% FBS-containing DMEM (Nacalai Tesque, Inc., Cat. No.: 08459-64), and cells with passage number 10 were used. TIG120 was cultured in 10% FBS-containing MEM medium (Nacalai Tesque, Inc., Cat. No.: 21442-25) and cells within PDL30 were used. The obtained alveolar organoids were dissociated using 0.1% tripsin-EDTA at 37°C for 15 minutes, and then washed twice with 1% BSA-containing PBS. After the washing, the cells were immunostained with anti-EpCAM-APC-antibody (Miltenyi Biotec, Cat. No.: 130-113-263). After the staining, SFTPC-GFP positive cells/EPCAM positive cells were analyzed using a flow cytometer (FACS). The alveolar organoids were observed using the fluorescent microscope. In addition, alveolar organoids were prepared in the same manner except that 604A1 cell line was used as iPSC cell line. The results thereof are shown in FIG. 6.
Reference 14: Korogi, Y, Gotoh, S., Ikeo, S., Yamamoto, Y, Sone, N., Tamai, K., Konishi, S., Nagasaki, T., Matsumoto, H., Ito, I., et al. (2019). In Vitro Disease Modeling of Hermansky-Pudlak Syndrome Type 2 Using Human Induced Pluripotent Stem Cell-Derived Alveolar Organoids. Stem Cell Reports 12, 431-440, which is herein incorporated by reference.

**[Table 3]**

| | Alveolarization medium |
|---|---|
| Basal medium | Ham' s F12 |
| | B27 supplement (1 %) |
| | BSA (0.25 %) |
| | HEPES (15 mmol/1) |
| | CaCl₂ (0.8 mmol/l) |
| | ITS premix (0.1 %) |
| | Penicillin / Streptomycin (50 U/ml) |
| Compound/cytokine | Dexamethasone (50 nmol/I) |
| | IBMX (100 *µ*mol/l) |
| | KGF (10 ng/ml) |
| | 8-Br-cAMP (100 *µ*mol/l) |
| | Y-27632 (Day 0 of culture: 10 µmol/l, add daily in the case of FF-AOs) |

FIG. 6 shows graphs showing the results of examination of alveolar organoids. In FIG. 6, (A) shows the fluorescent image of alveolar organoids, (B) shows the analysis result of flow cytometry, and (C) shows the proportion of SFTPC-GFP positive cells in EpCAM positive cells in alveolar organoids. As shown in (A) of FIG. 6, when iMESs and the lung progenitor cells were co-cultured, spheroids containing SFTPC-GFP positive cells were formed, and it was observed that the lung progenitor cells were differentiated into alveolar epithelial cells. In addition, although it is not shown, spheroids that contain SFTPC-GFP positive cells were formed in the same manner as that of when 604A1 cell line was used. As shown in (B) and (C) of FIG. 6, the proportion of SFTPC-GFP positive cells induced by iMESs was comparable to HFLFs used as feeder cells. On the other hand, no SFTPC-GFP positive cells were induced in HDFs.

Next, the cells constituting the alveolar organoids were examined. Specifically, the alveolar organoids were fixed using 4% paraformaldehyde-containing PBS for 20 minutes and then incubated overnight (about 8 hours) in 30% sucrose-containing PBS. After the incubation, the alveolar organoids were embedded in OCT compounds (Sakura Finetek Japan Co., Ltd, Cat. No.: 4583) and frozen using liquid-nitrogen. The frozen alveolar organoids were sliced into 10 µm thick sections and then applied to slides. The resulting sections were permeabilized for 15 minutes using 0.2% TRITON^{®} X-100-containing PBS. After the permeabilization, blocking was carried out for 30 minutes using PBS that contains 5% normal donkey serum (EMD, Millipore) and 1% BSA. After the blocking, primary antibodies and secondary antibodies against EPCAM, VIM, Pro-SFTPC, ABCA3, GFP, Mature-GFP, SFTPB, PDPN, and HT1-56 were used for staining. To the secondary antibody solution, Hoechst-33342 (Dojindo Laboratories, Cat. No.: H342) was added to label the nuclei of cells. The stained sections were observed using the fluorescent microscope. In addition, alveolar organoids were prepared in the same manner except that 604A1 cell line was used as PSC cell line.

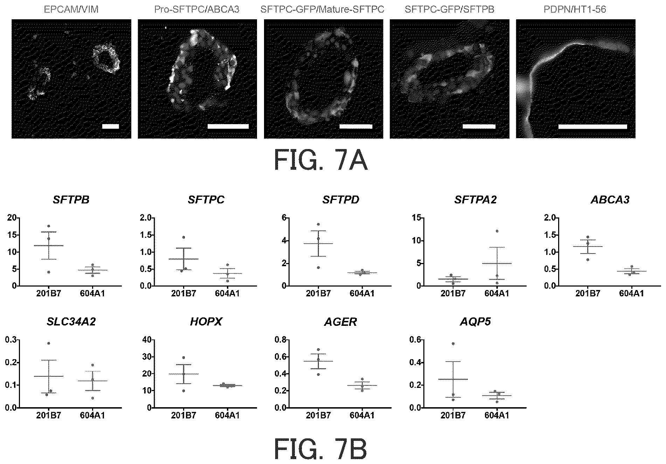

Also, alveolar organoids formed using 201B7 cell line and 604A1 cell line were used and the genes to be measured were SFTPB, SFTPC, SFTPD, SFTPA2, ABCA3, SLC34A2, HOPX, AGER, and AQP5. For exogenous control, human fetal lung RNA (Agilent Technologies; #540177, lot 0006055802) at 17, 18, or 22 weeks of gestation were used to quantify the relative expression levels. Except for this, the expressions of these genes in the alveolar organoids were examined in the same manner as that in Example 1(1). The results thereof are shown in FIG. 7.

FIG. 7 shows photographs and graphs showing the expressions of various cell markers in the alveolar organoids. In FIG. 7, (A) shows a fluorescence image of the alveolar organoid, and (B) shows the relative expression level of the genes. In each graph in (B) of FIG. 7, the horizontal axis indicates iPSC line, and the vertical axis indicates the relative expression level of each gene. As shown in (A) of FIG. 7, in the alveolar organoids that contain SFTPC-GFP positive cells, VIM positive iMESs spread throughout the alveolar organoids. Also, as shown in (A) and (B) of FIG. 7, in the alveolar organoids, Pro-SFTPC, ABCA3, SFTPC-GFP, and mature-SFTPC, which are type II alveolar epithelial cell markers, were detected in cuboidal cells. PDPN positive and HT1-56 positive squamous cells, which are type I alveolar epithelial cell markers, were also observed in the alveolar organoids. As shown in (B) of FIG. 7, in the alveolar organoids (iMES-AO) formed by co-culturing with iMESs, the type I alveolar epithelial cell markers and the type II alveolar epithelial cell markers were detected. This showed that the alveolar organoid could be formed, when the iPSC-derived lung progenitor cells and iMESs were co-cultured three-dimensionally.

From the above, it was verified that lung mesenchymal cells can be induced by the production method of the present disclosure, and that alveolar organoids can be formed by co-culturing the lung mesenchymal cells and the lung progenitor cells. In addition, since alveolar organoids can be formed by using the lung mesenchymal cells obtained by the production method of the present disclosure, it was verified that the lung mesenchymal cells (iMESs) can be used as feeder cells instead of HFLFs.

### [Example 2]

It was examined whether alveolar organoids can be formed using iMESs induced from iPSCs derived from other cells. The expression profile of iMESs before and after the culture was analyzed.

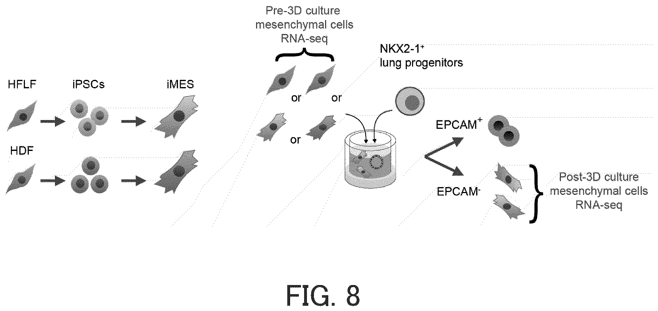

As shown in FIG. 8, iPSCs were induced from different cells to examine whether alveolar organoids can be formed using iMESs derived from the different cells. That is, it was examined whether the induced lung mesenchymal cells can be used as feeder cells regardless of the origin of the pluripotent cells.

### (1) Induction of iPSC

iPSCs were induced from HFLFs and HDFs used in Example 1. First, HFLF-iPSCs (HFAs) were established from HFLFs (17.5 weeks gestation, DV Biologics LLC, Cat. No.: PP002-F-1349, lot 121109VA). To 1×10⁶ HFLFs, an episomal vector mix for human iPSC production (Takara, Cat. No.: 3673) that contains cDNA of OCT3/4, SOX2, KLF4, L-MYC, LIN28, mp53-DD, and EBNA1 was introduced by electroporation. 5×10⁴ cells out of the introduced cells were seeded in wells of a 6-well plate that contains 10 % FBS-containing DMEM (day 0 of culture). The medium was replaced with 10% FBS-containing DMEM on day 1, day 3, and day 5 of culture. On day 6 of culture, the medium of each well was replaced with StemFit AK02N (Ajinomoto Co., Inc., Cat. No.: AJ100). The obtained iPSC colonies were picked up and seeded in wells of a 12-well plate that contains StemFit AK02N and iMatrix-511 (TAKARABIO INC., Cat. No.: 892021) (0.25 µg/cm²). The resulting iPSCs were maintained and passaged with StemFit AK02N. After several passages, the medium of each well was replaced with mTeSR Plus (STEMCELL Technologies Cat. No.: ST-05825 or ST-100-0276), and then iPSCs (HFLF-iPSC) were used to induce iMESs.

HDF-iPSCs (GC23) were established in a feeder cell-dependent manner using the episomal vector for human iPSC production (short hairpin RNA (mp53-DD) for OCT3/4, SOX2, KLF4, L-MYC, LIN28, p53) from HDFs (TIG120) according to Reference 14. The resulting HDF-iPSCs were then grown and frozen. Also, after thawing of frozen cells, prior to induction into iMESs, HDF-iPSCs were maintained and passaged in a feeder cell-free manner using a mTeSR Plus medium.

Regarding HFLFs, HFLF-iPSCs (HFA), HDFs (TIG120), and HDF-iPSCs (GC23), 16 loci of short tandem repeats (Tables 4 below) of HFLFs, HFLF-iPSCs (HFA), HDFs, and HDF-iPSCs (GC23) were examined using POWERPLEX^{®} 16 HS System (Promega Corporation) to check genome identity with the parent cell line in order to exclude mix-up and cross-contamination of cells under operation. The tandem repeats of the loci were perfectly identical between iPSCs and the corresponding parental fibroblasts. Although it is not shown, HFLF-iPSCs and HDF-iPSCs expressed undifferentiated markers (Nanog, OCT3/4), exhibited no abnormal karyotypes, and showed ability to be differentiated into three primary germ layers (ectoderm, mesoderm, and endoderm). Thus, iMESs were prepared using HFLF-iPSCs (HFA) and HDF-iPSCs (GC23).

**[Table 4]**

| **Locus** | **HFLF** | | **HFA iPSCs** | | **TIG120** | | **GC23 iPSCs** | |
|---|---|---|---|---|---|---|---|---|
| D3S1358 | 15 | | 15 | | 14 | 15 | 14 | 15 |
| TH01 | 7 | 8 | 7 | 8 | 9 | 9.3 | 9 | 9.3 |
| D21S11 | 30 | | 30 | | 30 | 32.2 | 30 | 32. 2 |
| D18S51 | 12 | 15 | 12 | 15 | 14 | 15 | 14 | 15 |
| Penta_E | 12 | 17 | 12 | 17 | 5 | 21 | 5 | 21 |
| D5S818 | 7 | 12 | 7 | 12 | 9 | 11 | 9 | 11 |
| D13S317 | 8 | 12 | 8 | 12 | 10 | | 10 | |
| D7S820 | 11 | | 11 | | 10 | 12 | 10 | 12 |
| D16S539 | 9 | 12 | 9 | 12 | 12 | | 12 | |
| CSF1P0 | 11 | 12 | 11 | 12 | 12 | | 12 | |
| Penta_D | 10 | 14 | 10 | 14 | 9 | 10 | 9 | 10 |
| AMEL | X | | X | | X | | X | |
| vWA | 14 | 15 | 14 | 15 | 14 | 20 | 14 | 20 |
| D8S1179 | 12 | 13 | 12 | 13 | 13 | 14 | 13 | 14 |
| TPOX | 11 | 12 | 11 | 12 | 8 | 11 | 8 | 11 |
| FGA | 19 | 23 | 19 | 23 | 21 | 23 | 21 | 23 |

### (2) Induction of Lung Mesenchymal Cells (iMESs)

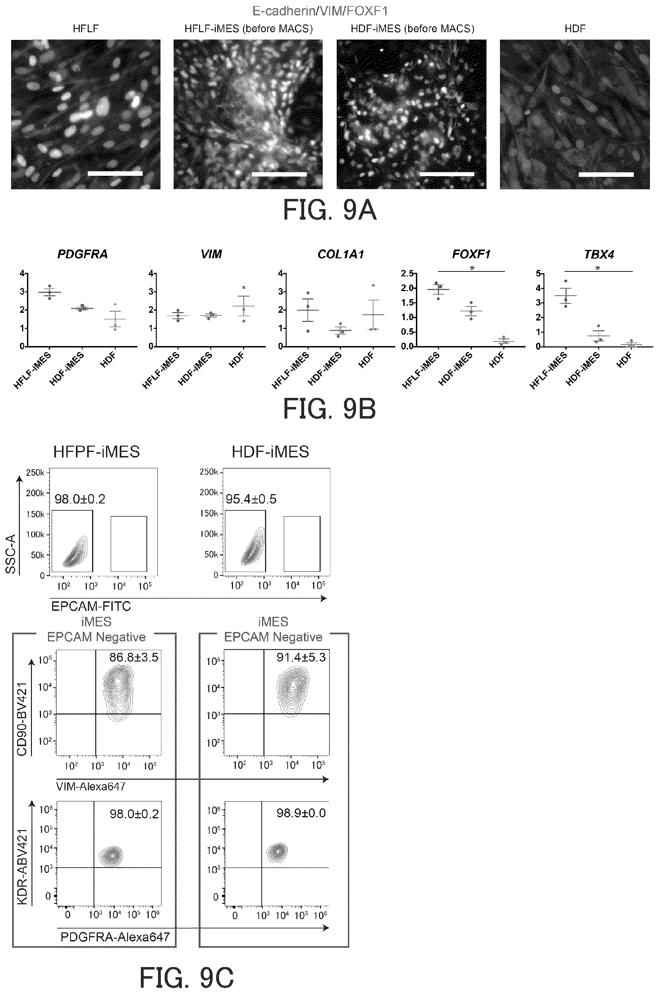

The induction from iPSCs to iMESs was carried out in the same manner as that in Example 1(1) above, except that HFLF-iPSCs (HFA) and HDF-iPSCs (GC23) were used as iPSCs. The obtained iMESs, HFLFs, and HDFs were stained with E-Cadherin, Vimentin (VIM), and FOXF1 in the same manner as that in Example 1(1) and observed by a fluorescent microscope. In addition, mRNA of PFGFRA, VIM, COL1A1, FOXF1, and TBX4 were quantified using HFLFs as an exogenous control in the same manner as that in Example 1(1), except that the obtained iMESs and HDFs were used. Then, the expressions of VIM, THY1, PDGFRA, and KDR were examined by flow cytometry with respect to the obtained iMESs in the same manner as that in Example 1(1). The results thereof are shown in FIG. 9.

FIG. 9 shows photographs and graphs relating to the expressions of iMES markers. In FIG. 9, (A) shows photographs showing fluorescence images of the cells, (B) shows the gene expressions of the cells, and (C) shows the analysis results of flow cytometry. (A) of FIG. 9 shows that VIM and FOXF1 are expressed also in iMESs derived from HFLF-iPSCs (HFA) and HDF-iPSCs (GC23) on a protein-level basis. As shown in (B) of FIG. 9, as in Example 1(1), it was observed that both HFLF-iPSCs (HFA) and HDF-iPSCs (GC23)-derived iMESs were differentiated into iMESs that express VIM, THY1, PDGFRA, and KDR. Furthermore, as in Example 1(1), the expression of FOXF1 was high in HFLFs and iMESs, but almost no expression was observed in HDFs.

### (3) Formation of Alveolar Organoid

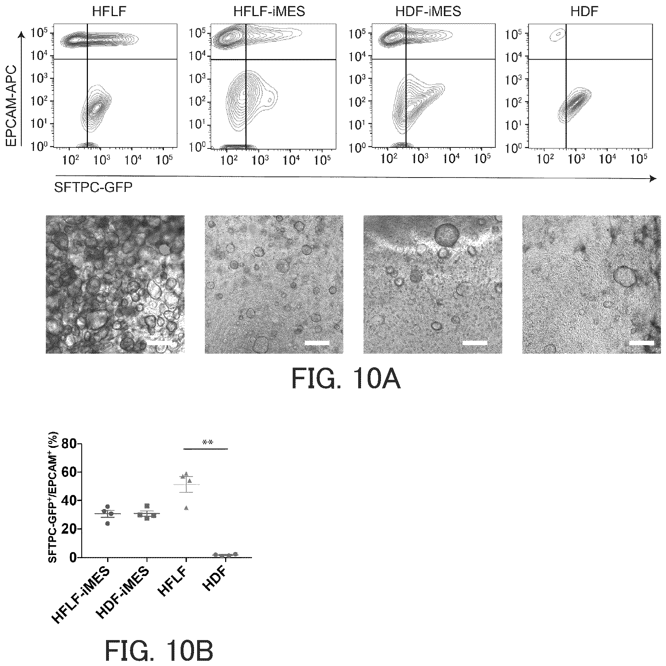

Using the obtained iMESs, HFLFs, and HDFs, alveolar organoids were formed in the same manner as that in Example 1(3). The alveolar organoids were observed using the fluorescent microscope. In the same manner as that in Example 1(3), the cells constituting the alveolar organoids were dissociated, and the obtained cells were analyzed for SFTPC-GFP positive cells/EPCAM positive cells. The results thereof are shown in FIG. 10.

FIG. 10 shows graphs and photographs relating to the organoid formation ability. In FIG. 10, (A) shows the analysis result of flow cytometry, and (B) shows the proportion of SFTPC-GFP positive cells in EpCAM positive cells in alveolar organoids. As shown in (A) and (B) of FIGS. 10, SFTPC-GFP positive cells were induced in both iMESs derived from HFLF-iPSCs (HFA) and iMESs derived from HDF-iPSCs (GC23), showing that alveolar epithelial cells could be induced by using these cells.

### (4) Transcriptome Analysis of iMES

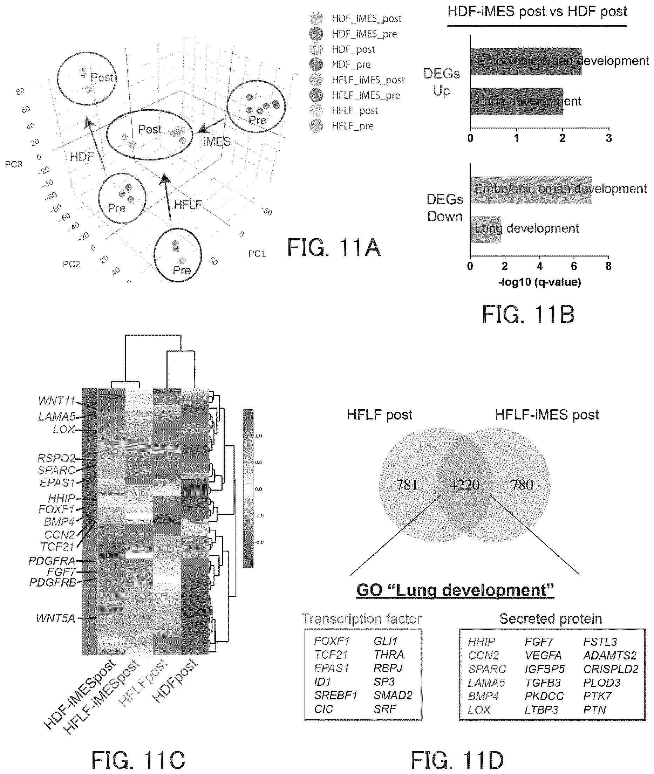

HFLFs, HFLF-iPSC-derived iMESs, HDF-iPSC-derived iMESs, and HDFs before and after alveolar organoid formation assays were subjected to RNA-Seq analysis to analyze the factors that function as feeder cells in alveolar organoid formation. Specifically, the total RNA was extracted using an RNA extraction kit (RNeasy micro kit, Qiagen) according to the attached protocol. For the obtained RNA, libraries of the samples were prepared using a TruSeq Stranded mRNA Library Prep Kit (Illumina, Inc.). The resulting libraries were sequenced with 100 bp paired-end reads using NovaSeq 6000 (Illumina, Inc.). For FASTQ raw data, after trimming using software (fastp 0.20.1, https://github.com/OpenGene/fastp#install-with-bioconda, Reference 15), software (SortMeRna 2.1b, https://github.com/biocore/sortmerna, Reference 16) was used to exclude rRNA, tRNA, snRNA, snoRNA, Mt_rRNA, and Mt_tRNA. The preprocessed data were aligned to GRCh38 using software (STAR 2.7.6a, https://github.com/alexdobin/STAR, Reference 17). The resulting alignment data and software (RSEM 1.3.3, https://github.com/deweylab/RSEM, Reference 18) were used to calculate transcripts per million (TPM) and read count. These data were imported into R 4.1.1 (http://www.R-project.org) using software (tximport 1.20.0, https://github.com/mikelove/tximport, Reference 19). Low-expression genes with an average read number in samples of the imported data of less than 1 were excluded as being lower than the detection limit from the analysis. After the exclusion, DEGs (Reference 21) were identified using software (DESeq2 1.32.0, https://github.com/mikelove/DESeq2, Reference 20). Then, pre-ranked GSEA was carried out using genes sorted in the order of p-values calculated by DESeq2. GO enrichment analysis was carried out using software (clusterProfiler 4.0.5, https://github.com/YuLab-SMU/clusterProfiler, Reference 22) and org.Hs.eg.db 3.13.0 (https://anaconda.org/bioconda/bioconductor-org.hs.eg.db). The results thereof are shown in FIG. 11.
Reference 15: Chen, S., Zhou, Y, Chen, Y, and Gu, J. (2018). fastp: an ultra-fast all-in-one FASTQ preprocessor. Bioinformatics 34, i884-i890, which is herein incorporated by reference.
Reference 16 : Kopylova, E., Noe, L., and Touzet, H. (2012). SortMeRNA: fast and accurate filtering of ribosomal RNAs in metatranscriptomic data. Bioinformatics 28, 3211-3217, which is herein incorporated by reference.
Reference 17: Dobin, A., Davis, C.A., Schlesinger, F., Drenkow, J., Zaleski, C., Jha, S., Batut, P., Chaisson, M., and Gingeras, T.R. (2013). STAR: ultrafast universal RNA-seq aligner. Bioinformatics 29, 15-21, which is herein incorporated by reference.
Reference 18: Li, B., and Dewey, C.N. (2011). RSEM: accurate transcript quantification from RNA-Seq data with or without a reference genome. BMC Bioinformatics 12, 323, which is herein incorporated by reference.
Reference 19: Soneson, C., Love, M.I., and Robinson, M.D. (2015). Differential analyses for RNA-seq: transcript-level estimates improve gene-level inferences. F1000Res 4, 1521, which is herein incorporated by reference.
Reference 20: Subramanian, A., Tamayo, P., Mootha, V.K., Mukherjee, S., Ebert, B.L., Gillette, M.A., Paulovich, A., Pomeroy, S.L., Golub, T.R., Lander, E.S., et al. (2005). Gene set enrichment analysis: a knowledge-based approach for interpreting genome-wide expression profiles. Proc Natl Acad Sci U S A 102, 15545-15550, which is herein incorporated by reference.
Reference 21: Love, M.I., Huber, W., and Anders, S. (2014). Moderated estimation of fold change and dispersion for RNA-seq data with DESeq2. Genome Biol 15, 550, which is herein incorporated by reference.
Reference 22: Wu, T., Hu, E., Xu, S., Chen, M., Guo, P., Dai, Z., Feng, T., Zhou, L., Tang, W., Zhan, L., et al. (2021). clusterProfiler 4.0: A universal enrichment tool for interpreting omics data. The Innovation 2, which is herein incorporated by reference.

FIG. 11 shows diagrams showing the analysis results of RNA-Seq. As shown in (A) of FIG. 11, in the principal component analysis (PCA) of the transcriptome of RNA-seq, clusters of the respective conditions were well separated, and the transcriptome of iMESs after alveolar organoid formation (hereinafter referred to as "3D culture") and the transcriptome of HFLFs after 3D culture were plotted close to each other. HDFs after 3D culture were separated from iMESs and HFLFs after 3D culture. Gene expression often depends on medium composition and culture conditions (e.g., 2D or 3D). Therefore, in order to clarify the factors contributing to the alveolar organoid formation in iMESs, gene expression was analyzed using the analysis results of samples after 3D culture paired with the parent cell lines (HDF-iMES-paired HDF, HFLF-iMES-pairwd HFLF). As shown in (C) of FIG. 11, in GO enrichment analysis, "Embryonic development" and "Lung development" were enriched between HDF-iMESs and HDFs after 3D culture. DEGs annotated to "Lung development" were extracted and heat maps were created in four groupings: HDF-iMESs, HDFs, HFLF-iMESs, and HFLFs. WNT5A, FGF7, and PDGFRA, which are known as key factors in type II alveolar epithelial cells, were unexpectedly increased in expression in HDFs, as shown in (C) of FIG. 11. On the other hand, in iMESs, the expression levels of secreted proteins such as RSPO2, WNT11, CCN2, SPARC, BMP4, HHIP, LAMA5, LOX were increased. The expression levels of transcription factors (TF) such as FOXF1 and TCF21 were higher in HFLF-iMESs, HDF-iMESs, and HFLFs than those in HDFs. This suggested that iMES had the characteristics of lung fibroblast. While EPAS1 was a common gene to HFLF-iMESs and HFLFs, the EPAS1 expression level was higher in HDFs than that in HFLFs, suggesting that EPAS1 was not a marker specific to lung mesenchymal cells. Next, the top 5000 genes of HFLF-iMESs and HFLFs after 3D culture were picked up, and a Venn diagram was drawn. As shown in (D) of FIG. 11, 4220 genes were in common, and "Lung development" was enriched in these genes (FDR q - value = 0.001). HHIP, CCN2, SPARC, BMP4, LAMA5, and LOX were again included in the genes annotated to "Lung development", suggesting that these genes were key factors in alveolar organoid formation. In addition, transcription factors, FOXF1, TCF21, and EPAS1 annotated to "Lung development" were also included, suggesting that they might be markers of lung fibroblasts.

### (5) Functional analysis of RSPO2 and RSPO3 in iMES

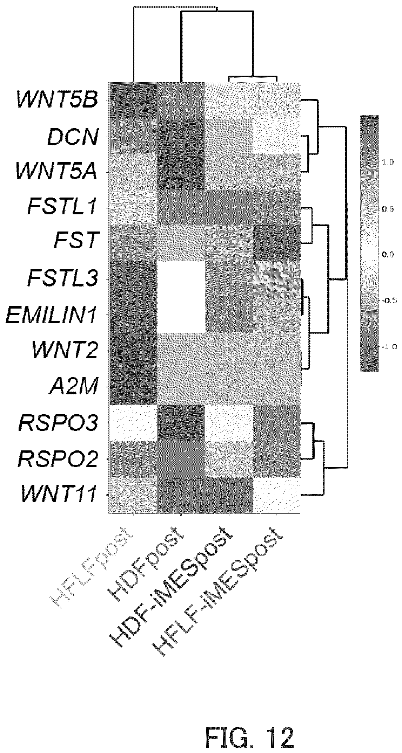

Based on the findings that Wnt ligands, antagonists of TGFβ family ligands, and the like contribute to the differentiation of type II alveolar epithelial cells, the inventors compared the expressions of Wnt ligands after 3D culture in the RNA-seq data. The results thereof are shown in FIG. 12 and Table 5 below.

**[Table 5]**

| Gene symbol | Post 3D culture HFLF-iMES | Post 3D culture HDF-iMES | Post 3D culture HFLF | Post 3D culture HDF |
|---|---|---|---|---|
| A2M | 1.6 | 2.5 | 2440.8 | 14.3 |
| DCN | 5772.4 | 4090. 6 | 2557.2 | 16111.3 |
| EMILIN1 | 76.9 | 36.2 | 509.4 | 182.2 |
| FST | 15.3 | 35.5 | 116.5 | 84.5 |
| FSTL1 | 364.3 | 331.9 | 1412.4 | 1603.1 |
| FSTL3 | 55.9 | 55. 8 | 174.9 | 87.7 |
| RSP02 | 176.4 | 141.7 | 14.1 | 0.3 |
| RSP03 | 59.7 | 37.5 | 39.3 | 0.7 |
| WNT11 | 25.9 | 58.9 | 19.2 | 0.8 |
| WNT2 | 0.3 | 0.6 | 67.9 | 0.9 |
| WNT5A | 9.4 | 17.3 | 43.8 | 559.2 |
| WNT5B | 16.3 | 22.9 | 12.3 | 30.2 |

FIG. 12 and Table 5 show the expression levels (TPM) of Wnt ligands and TGFβ antagonists. As shown in FIG. 12 and Table 5, after 3D culture, the expression levels of RSPO2 and RSPO3 were higher in HFLF-iMESs, HDF-iMESs, and HFLFs than those in HDFs. Therefore, the roles of RSPO2 and RSPO3 in alveolar organoid formation was examined. Examined were the roles of TGFβ antagonists (FST, FSTL1, FSTL3, DCN), which were lower than HFLFs and HDFs in terms of their relative expression levels, but showed adequate expression.

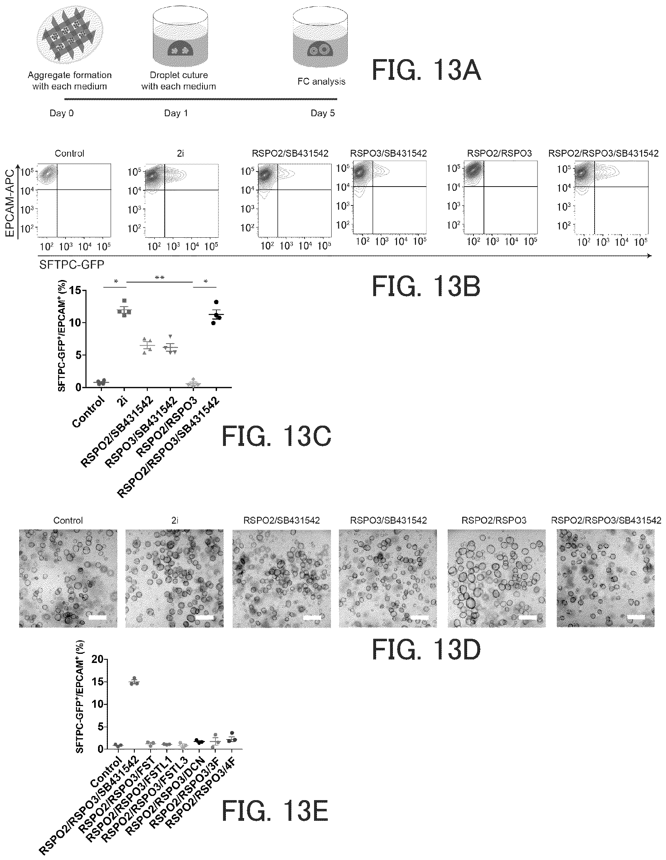

Next, as shown in (A) of FIG. 13, 2.0×10⁵ isolated CPM positive lung progenitor cells and the respective factors (Y-27632 10 µmol/L, CHIR99021 3 µmol/L, RSPO2 200 ng/mL, RSPO3 200 ng/mL, SB431542 10 µmol/L, FST 200 ng/mL, FSTL1 200 ng/mL, FSTL3 200 ng/mL, DCN 200 ng/mL) were seeded in a 96-well plate (Corning Incorporated, Cat. No.: 4446) coated with poly(2-hydroxyethyl methacrylate) (Sigma-Aldrich Co. LLC, Cat. No.: 192066) and cultured at 37°C with 5% CO₂ for 24 hours to form aggregates of cells (spheroids). After centrifuging the resulting spheroids, the spheroid-containing pellets were gently resuspended in 20 µL of pre-cooled Matrigel and introduced into wells of a 24-well plate (Greiner Bio-One, Cat. No.: 662160). After the introduction, the cells were incubated at 37°C for 20 minutes, and 500 µL of the alveolarization medium supplemented with each factor was added onto the spheroid embedded in the Matrigel. The medium was changed every 2 days. Five days after the spheroids were formed, the cells were dissociated using 0.1% tripsin-EDTA at 37°C for 15 minutes, and then washed twice with 1% BSA-containing PBS. The washed cells were immunostained with anti EPCAM-APC antibody. Subsequently, the proportion of SFTPC-GFP positive cells in EpCAM positive cells was assessed by flow cytometry.

FIG. 13 shows a diagram showing the culture method and graphs and photographs showing the analysis results of SFTPC-GFP positive cells. In FIG. 13, (A) shows the culture method, (B) shows the analysis results of flow cytometry, (C) shows the proportion of SFTPC-GFP positive cells in EpCAM positive cells, (D) shows GFP positive cells in the wells, and (E) shows the proportion of SFTPC-GFP positive cells in EpCAM positive cells. As shown in (B), (C), and (D) of FIG. 13, GFP positive cells were observed by culture of 4 days under any of these conditions. In addition, as shown in (B) and (C) of FIG. 13, RSPO2/SB431542-added group, RSPO3/SB431542-added group, and RSPO2/RSPO3/SB431542-added group increased the proportion of SFTPC-GFP positive cells in EpCAM positive cells, and in particular, RSPO2/RSPO3/SB431542-added group increased the proportion of SFTPC-GFP positive cells in EpCAM positive cells as much as 2i (CHIR99021/SB431542). Also, as shown in (E) of FIG. 13, none of the antagonists of TGFβ family ligands increased the proportion of SFTPC-GFP positive cells in EpCAM positive cells. These results suggest that RSPO2 and RSPO3 of iMESs contributed to alveolar organoid formation, and that SB431542 contributed to alveolar organoid formation through another endogenous mechanism that replaces its roles in inhibiting the TGFβ pathway. As another endogenous mechanism, the following mechanism is presumed. That is, since SB431542 is an artificial low-molecular-weight compound, it is presumed that it induces type II alveolar epithelial cells by a mechanism other than inhibition of the TGFβ pathway in living organisms, and that iMES also induces type II alveolar epithelial cells by a mechanism other than inhibition of the TGFβ pathway. It is also presumed that iMESs secrete substances that inhibit the TGFβ pathway more potently than FST. The present invention, however, is not limited to the above presumption.

### (6) Type of Mesenchymal Cell

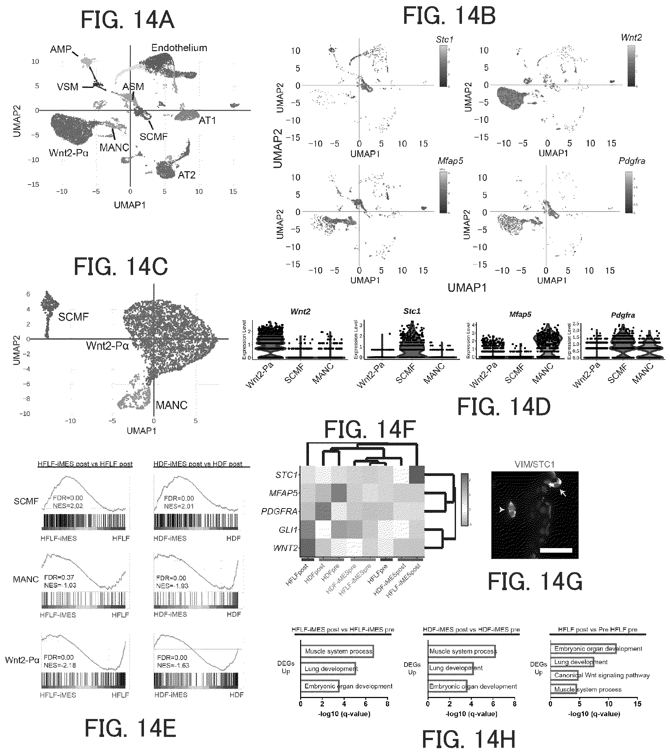

Different types of mesenchymal cells have been identified in mice (myofibrillar cells, mesenchymal alveolar niche cells, References 23 and 24). Therefore, the scRNA-seq published data in References 23 and 24 were analyzed to examine which types of mesenchymal cells iMES resembles. Specifically, three types of mesenchymal cells, Secondary crest myofibroblast (SCMF), Wnt2-Pα cells, and mesenchymal alveolar niche cell (MANC), were re-clustered and genes significantly increased in expression in the cell clusters of the respective lines were picked up. Next, the gene sets showing the characteristics of the clusters of the respective mesenchymal cells were constructed. Then, using software (biomaRt, https://github.com/grimbough/biomaRt, Reference 25), the murine genes were converted into the corresponding human genes, and then GSEA was performed using the scRNA-seq analysis data of iMESs, HFLFs and HDFs. Specifically, the scRNA-seq data was downloaded from GSE149563. Normalization of gene expression data, dimensionalization, and visualization of data were carried out using Seurat 4.0.5 and Plotly 4.9.4.1. The increased expression genes in the clusters were picked up with the FindAllMarkers of Seurat using Wilcoxon rank sum test with a cutoff of P < 0.05, and the genes expressed in more than 25% of the cells were extracted. After the extraction, the extracted murine genes were converted into human genes using biomaRt 2.48.3. The results thereof are shown in FIG. 14.
Reference 23: Zepp, J.A., Morley, M.P., Loebel, C., Kremp, M.M., Chaudhry, F.N., Basil, M.C., Leach, J.P., Liberti, D.C., Niethamer, T.K., Ying, Y, et al. (2021). Genomic, epigenomic, and biophysical cues controlling the emergence of the lung alveolus. Science 371, which is herein incorporated by reference.
Reference 24: Zepp, J.A., Zacharias, W.J., Frank, D.B., Cavanaugh, C.A., Zhou, S., Morley, M.P., and Morrisey, E.E. (2017). Distinct Mesenchymal Lineages and Niches Promote Epithelial Self-Renewal and Myofibrogenesis in the Lung. Cell 170, 1134-1148 e1110, which is herein incorporated by reference.
Reference 25: Durinck, S., Spellman, P.T., Birney, E., and Huber, W. (2009). Mapping identifiers for the integration of genomic datasets with the R/Bioconductor package biomaRt. Nat Protoc 4, 1184-1191, which is herein incorporated by reference.

FIG. 14 shows graphs showing the results of cluster analysis of mesenchymal cells. As shown in (A) to (C) of FIG. 14, markers specific to the mesenchymal cells were consistent with those in References 23 and 24. As shown in (D) of FIG. 14, Wnt2 showed high expression level in Wnt2-Pα, Stc1 showed high expression level in SCMF, and Mfap5 showed high expression level in MANC clusters. Also, as shown in (E) of FIG. 14, the SCMF gene set was enriched in iMESs more than in HFLFs and HDFs after 3D culture. On the other hand, as shown in (E) of FIG. 14, the gene set of MANC was enriched in HDFs, and the gene set of Wnt2-Pα was enriched in HFLFs. Further, as shown in (F) of FIG. 14, STC1 showed high expression level in iMESs, WNT2 showed high expression level in HFLFs, and MFAP5 showed high expression level in HDFs after 3D culture. Further, as shown in (G) of FIG. 14, in the immunofluorescent staining, after the alveolar organoids were formed, STC1 positive VIM positive cells were detected in iMESs, but some of the epithelial cells were also stained. As a result, "Muscle system process" was enriched in iMESs after 3D culture more than in iMESs before 3D culture, suggesting that the characteristics of mesenchymal cells of the muscular system (SCMF) were acquired during 3D culture. In addition, as shown in (H) of FIG. 14, "Canonical Wnt signaling pathway" was enriched in HFLFs after 3D culture more than in HFLFs before 3D culture, suggesting that the characteristics of Wnt2-Pα mesenchymal cells were acquired.

From the above, it was verified that alveolar organoids can be formed using iMESs, that iMESs contribute to the differentiation of alveolar epithelial cells by RSPO2 and/or RSPO3, and that the gene expression profile of iMES is similar to that of SCMF.

### [Example 3]

It was examined whether the alveolar epithelial cells can be maintained and cultured using iMESs.

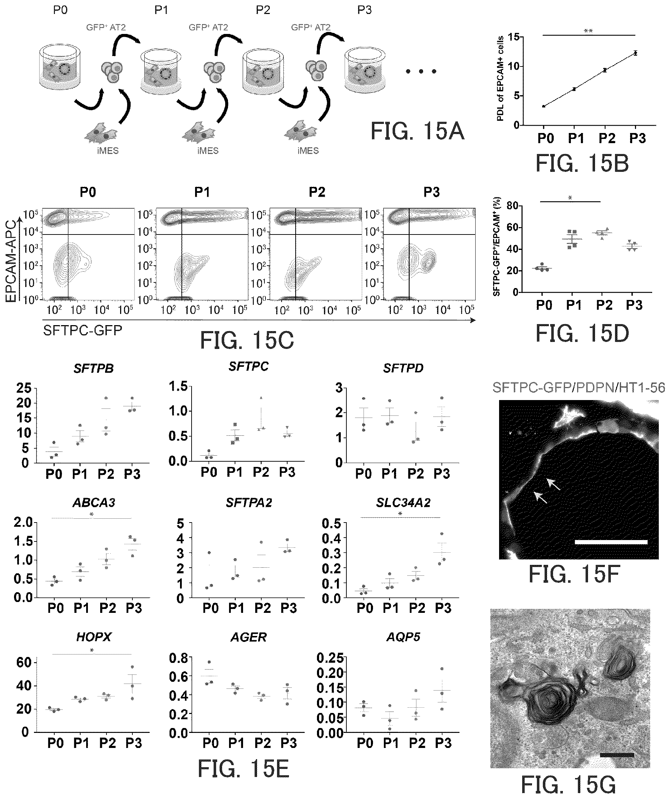

Alveolar organoids were formed in the same manner as that in Example 1(3). From the obtained alveolar organoids, SFTPC-GFP positive type II alveolar epithelial cells were fractionated, and it was examined whether they can be maintained and cultured in iMESs, which was performed, specifically, as shown in (A) of FIG. 15. First, the alveolar organoids obtained in the same manner as that in Example 1(3) were formed into single cells using 0.1% Trypsin-EDTA-containing PBS. Immunostaining was carried out using an anti EPCAM-APC antibody (Miltenyi Biotec, Cat No.: 130-113-263), and SFTPC-GFP+/EPCAM+ cells were harvested using FACS. After the harvest, 1×10⁵ harvested type II alveolar epithelial cells and 5×10⁵ iMESs were mixed into 100 µL of alveolarization medium supplemented with Y-27632 (10 µmol/L) and 100 µL of Matrigel. After the mixing, the resultant was 3D embedded in 12-well cell culture insert and cultured for 14 days (P0). In the above culture, the alveolarization medium was used. After the culture, SFTPC-GFP positive cells were harvested, the number of type II alveolar epithelial cells was counted, and the cells were cultured with iMESs under the same conditions (P1). The same passage was repeated (P2 to P3). The passage was performed every 2 weeks. P0 to P3 cells were analyzed for SFTPC-GFP positive cells/EPCAM positive cells using a flow cytometer (FACS) in the same manner as that in Example 1(3). The expressions of SFTPB, SFTPC, SFTPD, ABCA3, SFTPA2, SLC34A2, HOPX, AGER, and AQP5 were quantified for P0 to P3 cells in the same manner as that in Example 1(1). In addition, alveolar organoids were stained with primary antibodies and secondary antibodies against Mature-GFP, PDPN, and HT1-56 in the same manner as that in Example 1(3).

In order to examine whether the lamellar structure of type II alveolar epithelial cells was observed, observation was carried out by using the electron microscope. Specifically, small pieces of alveolar organoids obtained in the respective cultures were incubated overnight at 4°C in a fixative consisting of 2.5% glutaraldehyde, 4% paraformaldehyde, 1% tannic acid, and 0.1 mol/L phosphate buffer (pH 7.4). On the next day, the fixative was replaced with one without tannic acid. Specifically, washing with 0.1 mol/L phosphate buffer (pH7.4) for 20 minutes was carried out three times. After the washing, the mixture was fixed with 1% osmium tetraoxide for 2 hours, gradually dehydrated, and embedded in pure Epon as described above (Reference 12). After the embedding, ultrathin sections were stained with uranyl acetate and lead citrate and analyzed using a transmission electron microscope (JEOL Ltd.; JEM-1400). The results thereof are shown in FIG. 15.

FIG. 15 shows a diagram, graphs, and photographs showing the passage results of type II alveolar epithelial cells. As shown in (B) of FIG. 15, EpCAM positive cells increased linearly in P0 to P3. Also, as shown in (C) and (D) of FIG. 15, SFTPC-GFP positive cells increased at the first passage and significantly increased over P0 to P2, reaching a plateau. Furthermore, as shown in (E) of FIG. 15, ABCA3, SLC34A2 (type II alveolar epithelial cell markers), and HOPX (type II alveolar epithelial cell marker) were significantly increased over P0 to P3, showing that not only type II alveolar epithelial cells but also type I alveolar epithelial cells had matured. Furthermore, the expressions of SFTPB, SFTPD2, SFTPA2, AGER, and AQP5, which are markers of other alveolar epithelial cells, were maintained during passage, indicating that various epithelial cells forming alveolar organoids were maintained. As shown in (F) of FIG. 15, both SFTPC-GFP positive type II alveolar epithelial cells and PDPN positive HT1-56 positive type I alveolar epithelial cells were observed in the alveolar organoids of P2. As shown in (G) of FIG. 15, a lamellar bodies that are specific to type II alveolar epithelial cells were observed in the alveolar organoids. From the above, it was verified that iMESs are capable of maintenance-culturing type II alveolar epithelial cells, which are lung tissue stem cells, and are capable of inducing differentiation of various epithelial cells constituting alveolar organoids.

### [Example 4]

The ligand-target and ligand-receptor interactions between iMESs and alveolar epithelial cells were analyzed.

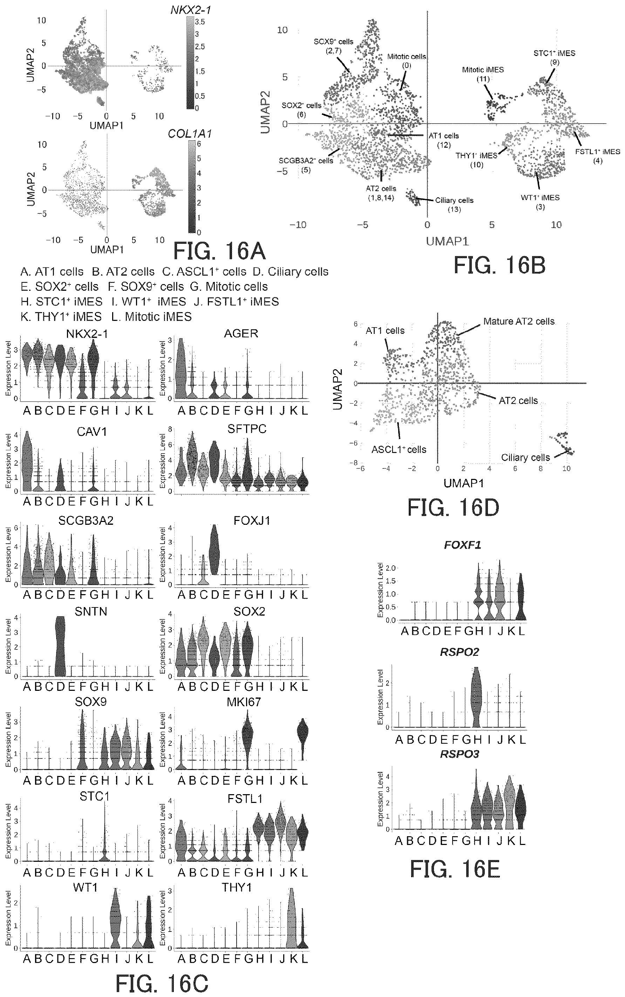

Alveolar organoids after P2 culture of Example 3 were used to analyze ligand-target and ligand-receptor interactions that are important for interaction with alveolar epithelial cells. Specifically, scRNA-seq analysis was performed on iMESs after P2 culture. Single-cell RNA libraries were prepared from iMESs, HFLFs, and HDFs using a 10×genomics Chromium device according to the attached protocol (Single Cell 3' Reagent Kits v3.1). The resulting libraries were sequenced using NovaSeq 6000 (Illumina, Inc.). The resulting readings were then mapped to GRCh38 and a counting matrix was created using Cell Ranger Software (Seurat 4.0.4, Reference 26) was used to process the obtained single-cell data. In the processing, dead and low-quality cells were excluded by deleting data of cells expressing the mitochondrial gene at 20% or more and less than 1.5%. Also, to exclude doublets of cells and low-quality cells, cells with UMI of 140,000 or more and less than 5,000 and with expression genes of less than 2,000 were also removed. The number of UMIs was then normalized with SCTransform. Then, the principal component (PC) analysis was performed on the resulting data using RunPCA of Seurat, and embedded in UMAP using RunUMAP of Seurat with 17 PCs and resolution 1. Plots of UMAP were visualized with Plotly 4.9.4.1 and plots of violin were drawn with Seurat. Analysis of ligand activity, predictions of active ligands, their target genes, and receptors were performed using software (nichenetr 1.0.0, Reference 27). Trajectory inference among epithelial cells, including type I alveolar epithelial cells, type II alveolar epithelial cells, ASCL1 positive cells, and ciliated cells, was performed using software (monocle3 1.0.0, Reference 28). The results thereof are shown in FIG. 16.
Reference 26: Hao, Y, Hao, S., Andersen-Nissen, E., Mauck, W.M., 3rd, Zheng, S., Butler, A., Lee, M.J., Wilk, A.J., Darby, C., Zager, M., et al. (2021). Integrated analysis of multimodal single-cell data. Cell 184, 3573-3587 e3529, which is herein incorporated by reference.
Reference 27: Browaeys, R., Saelens, W., and Saeys, Y (2020). NicheNet: modeling intercellular communication by linking ligands to target genes. Nat Methods 17, 159-162, which is herein incorporated by reference.
Reference 28: Cao, J., Spielmann, M., Qiu, X., Huang, X., Ibrahim, D.M., Hill, A.J., Zhang, F., Mundlos, S., Christiansen, L., Steemers, F.J., et al. (2019). The single-cell transcriptional landscape of mammalian organogenesis. Nature 566, 496-502, which is herein incorporated by reference.

FIG. 16 shows the cluster analysis results of the scRNA-seq analysis. As shown in (A) of FIG. 16, alveolar epithelial cells and iMESs were separated as they highly express NKX2-1 and COL1A1, respectively. Further, as shown in (B) of FIG. 16, as a result of the cluster analysis, the alveolar organoids after P2 culture were annotated to 15 clusters. The clusters were determined to be clusters of the following cells based on genes having high expression in the clusters. Specifically, as shown in (C) of FIG. 16, cluster 12 was considered to be type I alveolar epithelial cells due to higher expression ofAGER and CAV1. Clusters 1, 8, and 14 were considered to be type II alveolar epithelial cells due to higher expression of SFTPC. As to other epithelial cell clusters, cluster 5 was considered to be ASCL1 positive cells, cluster 2 and 7 were considered to be SOX9 positive cells, cluster 6 was considered to be SOX2 positive cells, and cluster 0 was considered to be cells during cell division due to the expression of MKI67. Cluster 13 showed high expression levels of FOXJ1, SNTN, and SFTPC, indicating that SFTPC positive distal tip cells were differentiated into ciliated cells. On the other hand, as shown in (D) of FIG. 16, iMESs were divided into five clusters. Specifically, as shown in (B) of FIG. 16, cluster 9 was considered to be STC1 positive iMESs, cluster 4 was considered to be FSTL1 positive iMESs, cluster 10 was considered to be THY1 positive iMESs, cluster 3 was considered to be WT1 positive iMESs, and cluster 11 was considered to be iMESs during cell division. Among genes of iMES transcriptome after 3D culture, FOXF1, RSPO2 and RSPO3 were examined. FOXF1 positive and RSPO3 positive iMESs were widely distributed in the clusters of the mesenchymal system. On the other hand, as shown in (E) of FIG. 16, RSPO2 was specifically expressed in STC1 positive iMESs.

Next, intercellular communication between iMESs and alveolar epithelial cells was analyzed using software (NicheNet 1.0.0, https://github.com/saeyslab/nichenetr, Reference 27). In the above analysis, gene sets of type I alveolar epithelial cells (AT1) and type II alveolar epithelial cells (AT2) were defined as summarized in Table 6 below. Representative genes for type I alveolar epithelial cells (AT1) and type II alveolar epithelial cells (AT2) were obtained from Lung Gene Expression Analysis Web Portal (Reference 29). Representative genes of lung genes listed by FindAllMarkers of Seurat (adjusted P value < 0.05) and common genes that were increased in expression in AT1 and AT2 clusters were extracted. Among these genes, NAMPT, HMGB1, and TGFB1 expressed in iMESs were ranked high in their ligand activity and broadly covered the typical genes of AT1. As to AT2 cells, it was speculated that TGFB2, HAS2, and CTF1 broadly regulate representative genes of AT2. These iMES-derived ligands (iMES-ligands) were expressed on various clusters of iMES, and therefore, various types of iMESs are considered to act in concert in the development process of alveolar epithelial cells. NicheNet was used to infer ligand-receptor interactions, considering only the ligand-receptor interactions described in the literature and published databases.
Reference 29: Du, Y, Ouyang, W., Kitzmiller, J.A., Guo, M., Zhao, S., Whitsett, J.A., and Xu, Y (2021). Lung Gene Expression Analysis Web Portal Version 3: Lung-at-a-Glance. Am J Respir Cell Mol Biol 64, 146-149, which is herein incorporated by reference.

**[Table 6]**

| AT1 | AT2 |
|---|---|
| AGER | ALCAM |
| AGRN | IRX2 |
| ALCAM | IRX3 |
| ANXA1 | LAMP3 |
| ASAH1 | MUC1 |
| BCAM | NAPSA |
| CD9 | NKX2-1 |
| CDH1 | NPC2 |
| DAG1 | SDC1 |
| EMP2 | SDC4 |
| GPRC5A | SFTPB |
| HOPX | SFTPC |
| ICAM1 | SLC34A2 |
| KRT7 | |
| LAMC2 | |
| LM07 | |
| NKX2-1 | |

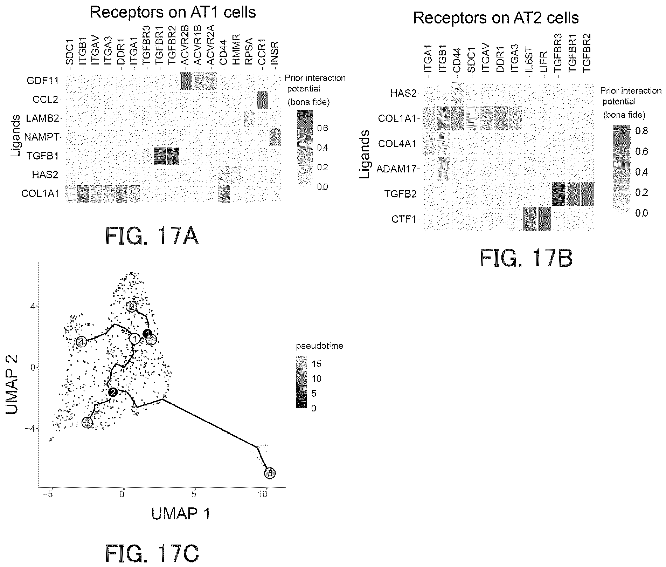

FIG. 17 shows the results showing the ligand-receptor interaction. As shown in (A) of FIG. 17, in type I alveolar epithelial cells, NAMPT and TGFB 1 interacted with INSR and TGFBR1/2/3, respectively. In addition, as shown in (B) of FIG. 17, in type II alveolar epithelial cells, TGFB2, HAS2, and CTF1 interacted with TGFBR1/2/3, CD44, and IL6ST/LIFR, respectively. These results support that ligands expressed in iMESs interact with alveolar epithelial cells and are involved in the expressions of target-marker genes. Further, as shown in (C) of FIG. 17, when the trajectory of differentiation of type I alveolar epithelial cells, type II alveolar epithelial cells, ASCL1 positive cells, and ciliated epithelial cells was presumed, it was suggested that type I alveolar epithelial cells, ASCL1 positive cells, and ciliated epithelial cells were cells that diverged around clusters of type II alveolar epithelial cells and were cells differentiated from type II alveolar epithelial cells, i.e., cells differentiated from type II alveolar epithelial cells.

### [Example 5]

It was examined whether lung mesenchymal cells can be efficiently induced from mesodermal cells by combining the mesenchymal cell-inducing factors with KGF and FGF10.

### (1) Induction of Lung Mesenchymal Cells

Mesenchymal cells were induced from mesodermal cells in the same manner as that in Example 1(1), except that any one of activin A (AA), KGF, BMP4, FGF2, and FGF10 was removed.

### (2) Induction of Lung Progenitor Cells

The lung progenitor cells were induced in the same manner as that in Example 1(2), except that B2-3 line was used as iPSCs.

### (3) Alveolar Organoid Formation Assay

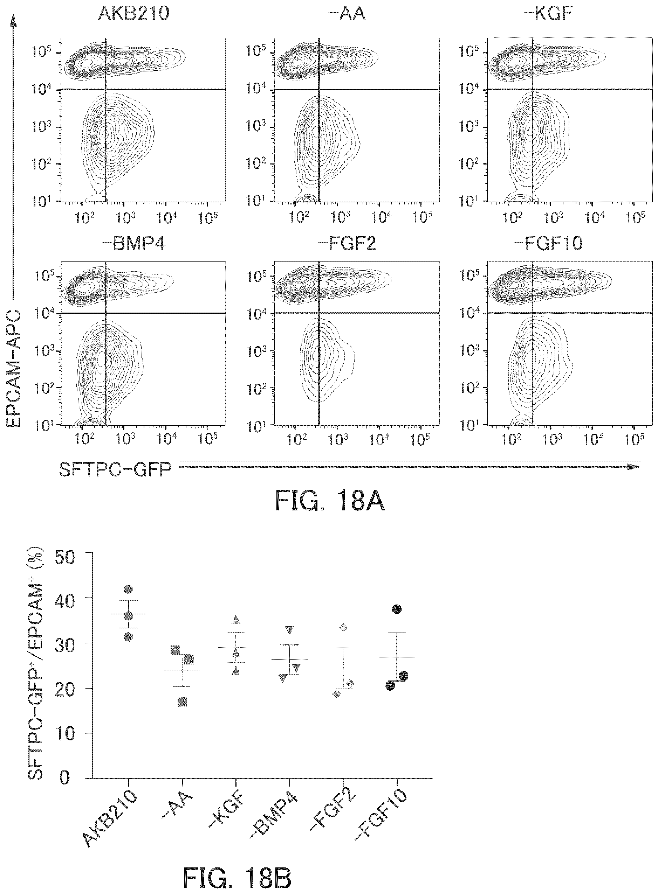

Alveolar organoids were formed in the same manner as that in Example 1(3), except that the lung mesenchymal cells of Example 5(1) and the lung progenitor cells of Example 5(2) were used. Then, the cells constituting the obtained alveolar organoids were isolated, and SFTPC-GFP positive cells/EPCAM positive cells were analyzed in the same manner as that in Example 1(3). The results thereof are shown in FIG. 18.

FIG. 18 shows graphs showing the results of SFTPC-GFP positive cells/EPCAM positive cells. In FIG. 18, (A) shows flow cytometry analysis, and (B) shows the proportion of SFTPC-GFP positive cells in alveolar organoids in EpCAM positive cells. In (B) of FIG. 18, the horizontal axis indicates the added factor (AKB210) or removed factor (-X), and the vertical axis indicates the proportion of SFTPC-GFP positive cells/EPCAM positive cells. AKB210 shows the case where all of activin A, KGF, BMP4, FGF2, and FGF10 are added. As shown in (A) and (B) of FIG. 18, the induction efficiency of alveolar epithelial cells was decreased upon removal of any of the factors, showing that the induction efficiency of lung mesenchymal cells could be efficiently induced by combining the mesenchymal cell-inducing factors with KGF and FGF 10.

While the present disclosure has been described above with reference to illustrative embodiments and examples, the present disclosure is by no means limited thereto. Various changes and modifications that may become apparent to those skilled in the art may be made in the configuration and specifics of the present disclosure without departing from the scope of the present disclosure.

This application claims priority from Japanese Patent Application No. 2022-014212 filed on February 1, 2022. The entire subject matter of the Japanese Patent Application is incorporated herein by reference.

### <Supplementary Notes>

Some or all of the above-described embodiments and examples may be described as the following supplementary notes, but are not limited thereto.

### <Method for Producing Lung Mesenchymal Cells>

### (Supplementary Note 1)

A method for producing lung mesenchymal cells, including:
culturing mesodermal cells in a presence of a mesenchymal cell-inducing factor, KGF, and FGF10 so as to induce differentiation of the mesodermal cells into lung mesenchymal cells.

### (Supplementary Note 2)

The method according to Supplementary Note 1, including:
enriching at least one type of cells, the one type of cells being selected from the group consisting of EpCAM cadherin negative lung mesenchymal cells and E-cadherin negative lung mesenchymal cells, from a cell population induced from the mesodermal cells.

### (Supplementary Note 3)

The method according to Supplementary Note 2, wherein
the enriching is enriching a cell population to include the at least one type of cells, the one type of cells being selected from the group consisting of EpCAM cadherin negative lung mesenchymal cells and E-cadherin negative lung mesenchymal cells, in a range of 50% or more.

### (Supplementary Note 4)

The method according to any one of Supplementary Notes 1 to 3, wherein
the lung mesenchymal cells express at least one factor selected from the group consisting of R-Spondin 2 (RSPO2) and R-Spondin 3 (RSPO3).

### (Supplementary Note 5)

The method according to any one of Supplementary Notes 1 to 4, wherein
the lung mesenchymal cells express a transcription factor selected from the group consisting of Forkhead box protein F1 (FOXF1), Transcription factor 21 (TCF21), T-Box Transcription Factor 4 (TBX4), and Odd-Skipped Related Transcription Factor (OSR1).

### (Supplementary Note 6)

The method according to any one of Supplementary Notes 1 to 3, wherein
the lung mesenchymal cells express at least one factor selected from the group consisting of R-Spondin 2 (RSPO2), R-Spondin 3 (RSPO3), Forkhead box protein F1 (FOXF1), Transcription factor 21 (TCF21), T-Box Transcription Factor 4 (TBX4), and Odd-Skipped Related Transcription Factor (OSR1).

### (Supplementary Note 7)

The method according to any one of Supplementary Notes 1 to 6, wherein
the lung mesenchymal cells do not express WNT2.

### (Supplementary Note 8)

The method according to any one of Supplementary Notes 1 to 7, wherein
the lung mesenchymal cells do not express T-box transcription factor T (TBXT).

### (Supplementary Note 9)

The method according to any one of Supplementary Notes 1 to 8, wherein
the lung mesenchymal cells express a fibroblast marker selected from the group consisting of Neural cell adhesion molecule (NCAM), Adipose differentiation-related protein (ADRP), Collagen, type I, alpha 1 (COL1A1), and Actin alpha 2 (ACTA2).

### (Supplementary Note 10)

The method according to any one of Supplementary Notes 1 to 9, wherein
the lung mesenchymal cell is positive for a mesenchymal cell marker selected from the group consisting of Vimentin (VIM), Thy-1 Cell Surface Antigen, CD90 (THY1), Platelet Derived Growth Factor Receptor α (PDGFRα), and Kinase Insert Domain Receptor (KDR).

### (Supplementary Note 11)

The method according to any one of Supplementary Notes 1 to 10, wherein
the mesenchymal cell-inducing factor includes a factor selected from the group consisting of activin A, FGF2, and BMP4.

### (Supplementary Note 12)

The method according to any one of Supplementary Notes 1 to 11, wherein
the lung mesenchymal cells can induce, in an alveolar organoid formation assay by co-culturing with at least one type of cells selected from the group consisting of lung progenitor cells, type I alveolar epithelial cells and type II alveolar epithelial cells from the lung progenitor cells.

### (Supplementary Note 13)

The method according to any one of Supplementary Notes 1 to 12, further including:
culturing, prior to the inducing the lung mesenchymal cells, pluripotent cells in a presence of a mesoderm-inducing factor so as to induce differentiation of the pluripotent cells into the mesodermal cells.

### (Supplementary Note 14)

The method according to Supplementary Note 13, wherein
the mesoderm-inducing factor includes a factor selected from the group consisting of a GSK3β inhibitor, activin A, and BMP4.

### (Supplementary Note 15)

The method according to Supplementary Note 14, wherein
the GSK3β inhibitor is CHIR99021.

### <Lung Mesenchymal Cells>

### (Supplementary Note 16)

A cell population including mesenchymal cells, including:
lung mesenchymal cells that express at least one factor selected from the group consisting of R-Spondin 2 (RSPO2) and R-Spondin 3 (RSPO3).

### (Supplementary Note 17)

The cell population according to Supplementary Note 16, wherein
the lung mesenchymal cells express a transcription factor selected from the group consisting of Forkhead box protein F1 (FOXF1), Transcription factor 21 (TCF21), T-Box Transcription Factor 4 (TBX4), and Odd-Skipped Related Transcription Factor (OSR1).

### (Supplementary Note 18)

The cell population according to Supplementary Note 16 or 17, wherein
the lung mesenchymal cells express RSPO2 and RSPO3.

### (Supplementary Note 19)

A cell population including mesenchymal cells, the cell population including:
lung mesenchymal cells that express at least one transcription factor selected from the group consisting of Forkhead box protein F1 (FOXF1), Transcription factor 21 (TCF21), T-Box Transcription Factor 4 (TBX4), and Odd-Skipped Related Transcription Factor (OSR1).

### (Supplementary Note 20)

The cell population according to any one of Supplementary Notes 16 to 19, wherein
the lung mesenchymal cells are at least one type of cells selected from the group consisting of EpCAM-cadherin negative cells and E-cadherin negative cells.

### (Supplementary Note 21)

The cell population according to Supplementary Note 20, wherein
a proportion (number of cells) of at least one type of cells selected from the group consisting of EpCAM-cadherin negative lung mesenchymal cells and E-cadherin negative lung mesenchymal cells in all of the cells of the cell population is 50% or more.

### (Supplementary Note 22)

The cell population according to any one of Supplementary Notes 16 to 21, wherein
the lung mesenchymal cells do not express Wnt2.

### (Supplementary Note 23)

The cell population according to any one of Supplementary Notes 16 to 22, wherein
the lung mesenchymal cells do not express T-box transcription factor T (TBXT).

### (Supplementary Note 24)

The cell population according to any one of Supplementary Notes 16 to 23, wherein
the lung mesenchymal cells express at least one fibroblast marker selected from the group consisting of Neural cell adhesion molecule (NCAM), Adipose differentiation-related protein (ADRP), Collagen, type I, alpha 1 (COL1A1), and Actin alpha 2 (ACTA2).

### (Supplementary Note 25)

The cell population according to any one of Supplementary Notes 16 to 24, wherein
the lung mesenchymal cell is positive for a mesenchymal cell marker selected from the group consisting of Vimentin (VIM), Thy-1 Cell Surface Antigen, CD90 (THY1), Platelet Derived Growth Factor Receptor α (PDGFRα), and Kinase Insert Domain Receptor (KDR).

### (Supplementary Note 26)

The cell population according to any one of Supplementary Notes 16 to 25, wherein
the lung mesenchymal cells can induce, in an alveolar organoid formation assay by co-culturing with at least one type of cells selected from the group consisting of lung progenitor cells, type I alveolar epithelial cells and type II alveolar epithelial cells from the lung progenitor cells.

### <Method for Producing Alveolar Epithelial Cells>

### (Supplementary Note 27)

A method for producing lung epithelial cells, including:
culturing lung progenitor cells in a presence of lung mesenchymal cells so as to induce differentiation of the lung progenitor cells into alveolar epithelial cells, wherein
the lung mesenchymal cells are the lung mesenchymal cells obtained by the method according to any one of Supplementary Notes 1 to 15 or the cell population according to any one of Supplementary Notes 16 to 26.

### (Supplementary Note 28)

The method according to Supplementary Note 27, wherein
the alveolar epithelial cells are at least one type of cells selected from the group consisting of type I alveolar epithelial cells and type II alveolar epithelial cells.

### (Supplementary Note 29)

The method according to Supplementary Note 27 or 28, wherein
the alveolar epithelial cells are alveolar epithelial cells constituting an alveolar organoid.

### (Supplementary Note 30)

The method according to any one of Supplementary Notes 27 to 29, wherein
the lung progenitor cells are at least one type of cells selected from the group consisting of NKX2-1 positive cells and CPM positive cells.

### (Supplementary Note 31)

The method according to any one of Supplementary Notes 27 to 30, wherein
the lung progenitor cells are cultured in the presence of the lung mesenchymal cells and an alveolar epithelial cell-inducing factor so as to induce differentiation of the lung progenitor cells into the alveolar epithelial cells.

### (Supplementary Note 32)

The method according to Supplementary Note 31, wherein
the alveolar epithelial cell-inducing factor is at least one factor selected from the group consisting of a Wnt promoter, a steroid agent, a cAMP derivative, a phosphodiesterase inhibitor, KGF, a GSK3β inhibitor, a TGFβ inhibitor, a ROCK inhibitor and FGF10.

### <Pharmaceutical Composition>

### (Supplementary Note 33)

A pharmaceutical composition including:
the lung mesenchymal cell obtained by the method according to any one of Supplementary Notes 1 to 15 or the cell population according to any one of Supplementary Notes 16 to 26; and
a pharmaceutically acceptable carrier.

### <Method for Maintenance-Culturing Type II Alveolar Epithelial Cells>

### (Supplementary Note 34)

A method for maintenance-culturing type II alveolar epithelial cells, including
culturing type II alveolar epithelial cells in a presence of lung mesenchymal cells to maintain, wherein
the lung mesenchymal cells are the lung mesenchymal cells obtained by the method according to any one of Supplementary Notes 1 to 15 or the cell population according to any one of Supplementary Notes 16 to 26.

### (Supplementary Note 35)

The method according to Supplementary Note 34, wherein
the type II alveolar epithelial cells are SFTPC positive cells.

### (Supplementary Note 36)

The method according to Supplementary Note 34 or 35, wherein
the type II alveolar epithelial cells are cultured in a presence of the lung mesenchymal cells and an alveolar epithelial cell-inducing factor to maintain the cells.

### (Supplementary Note 37)

The method according to Supplementary Note 36, wherein
the alveolar epithelial cell-inducing factor is at least one factor selected from the group consisting of a Wnt promoter, a steroid agent, a cAMP derivative, a phosphodiesterase inhibitor, KGF, a GSK3β inhibitor, a TGFβ inhibitor, a ROCK inhibitor, FGF10, and EGF.

### <Method for Expand-Culturing type II alveolar epithelial cells>

### (Supplementary Note 38)

A method for expand-culturing type II alveolar epithelial cells, including
culturing type II alveolar epithelial cells in a presence of lung mesenchymal cells to expand the cells, wherein
the lung mesenchymal cells are the lung mesenchymal cells obtained by the method according to any one of Supplementary Notes 1 to 15 or the cell population according to any one of Supplementary Notes 16 to 26.

### (Supplementary Note 39)

The method according to Supplementary Note 38, wherein
the type II alveolar epithelial cells are SFTPC positive cells.

### (Supplementary Note 40)

The method according to Supplementary Note 38 or 39, wherein
the type II alveolar epithelial cells are cultured in a presence of the lung mesenchymal cells and an alveolar epithelial cell-inducing factor to expand the cells.

### (Supplementary Note 41)

The method according to Supplementary Note 40, wherein
the alveolar epithelial cell-inducing factor is at least one factor selected from the group consisting of a Wnt promoter, a steroid agent, a cAMP derivative, a phosphodiesterase inhibitor, KGF, a GSK3β inhibitor, a TGFβ inhibitor, a ROCK inhibitor, FGF10, and EGF.

### <Medium>

### (Supplementary Note 42)

A medium for inducing lung mesenchymal cells from mesodermal cells, including:
a medium (basal medium);
a mesenchymal cell-inducing factor;
KGF; and
FGF10.

### (Supplementary Note 43)

The medium according to Supplementary Note 42, wherein
the mesenchymal cell-inducing factor includes a factor selected from the group consisting of activin A, FGF2, and BMP4.

### <Kit>

### (Supplementary Note 44)

A kit for inducing lung mesenchymal cells from mesodermal cells, the kit including:
a mesenchymal cell-inducing factor;
KGF; and
FGF10.

### (Supplementary Note 45)

The kit according to Supplementary Note 44, wherein
the mesenchymal cell-inducing factor includes a factor selected from the group consisting of activin A, FGF2, and BMP4.

### Industrial Applicability

As described above, according to the present disclosure, lung mesenchymal cells that can be used to produce alveolar organoids can be produced. Therefore, the present disclosure is extremely useful, for example, in the field of regenerative medicine, the field of cell medicine, and the like.

## Claims

1. A method for producing lung mesenchymal cells, comprising:
culturing mesodermal cells in a presence of a mesenchymal cell-inducing factor, KGF, and FGF10 so as to induce differentiation of the mesodermal cells into lung mesenchymal cells.

2. The method according to claim 1, comprising:
enriching at least one type of cells, the one type of cells being selected from the group consisting of EpCAM cadherin negative lung mesenchymal cells and E-cadherin negative lung mesenchymal cells, from a cell population induced from the mesodermal cells.

3. The method according to claim 2, wherein
the enriching is enriching a cell population to comprise the at least one type of cells, the one type of cells being selected from the group consisting of EpCAM cadherin negative lung mesenchymal cells and E-cadherin negative lung mesenchymal cells, in a range of 50% or more.

4. The method according to any one of claims 1 to 3, wherein
the lung mesenchymal cells express at least one factor selected from the group consisting of R-Spondin 2 (RSPO2), R-Spondin 3 (RSPO3), Forkhead box protein F1 (FOXF1), Transcription factor 21 (TCF21), T-Box Transcription Factor 4 (TBX4), and Odd-Skipped Related Transcription Factor (OSR1).

5. The method according to any one of claims 1 to 4, wherein
the mesenchymal cell-inducing factor comprises a factor selected from the group consisting of activin A, FGF2, and BMP4.

6. The method according to any one of claims 1 to 5, further comprising:
culturing, prior to the inducing the lung mesenchymal cells, pluripotent cells in a presence of a mesoderm-inducing factor so as to induce differentiation of the pluripotent cells into the mesodermal cells.

7. A cell population comprising mesenchymal cells, comprising:
lung mesenchymal cells that express at least one factor selected from the group consisting of R-Spondin 2 (RSPO2) and R-Spondin 3 (RSPO3).

8. A cell population comprising mesenchymal cells, the cell population comprising:
lung mesenchymal cells that express at least one transcription factor selected from the group consisting of Forkhead box protein F1 (FOXF1), Transcription factor 21 (TCF21), T-Box Transcription Factor 4 (TBX4), and Odd-Skipped Related Transcription Factor (OSR1).

9. The cell population according to claim 7 or 8, wherein
the lung mesenchymal cells are EpCAM negative.

10. The cell population according to any one of claims 7 to 9, wherein
the lung mesenchymal cells can induce, in an alveolar organoid formation assay by co-culturing with at least one type of cells selected from the group consisting of lung progenitor cells, type I alveolar epithelial cells, and type II alveolar epithelial cells, from the lung progenitor cells.

11. A method for producing lung epithelial cells, comprising:
culturing lung progenitor cells in a presence of lung mesenchymal cells so as to induce differentiation of the lung progenitor cells into alveolar epithelial cells, wherein
the lung mesenchymal cells are the lung mesenchymal cells obtained by the method according to any one of claims 1 to 6 or the cell population according to any one of claims 7 to 10.

12. A method for expand-culturing type II alveolar epithelial cells, comprising
culturing type II alveolar epithelial cells in a presence of lung mesenchymal cells to expand the cells, wherein
the lung mesenchymal cells are the lung mesenchymal cells obtained by the method according to any one of claims 1 to 6 and/or the cell population according to any one of claims 7 to 10.
